# EUROPEAN PATENT APPLICATION

(11) **EP 1 674 110 A1**
(43) Date of publication of application: **28.06.2006**
(21) Application number: 05112049.1
(22) Date of filing: 28.04.2000
(51) Int. Cl.: A61K 38/55, A61K 39/395, A61P 37/00

(54) **Inhibition of bradykinin release**

(30) Priority: 29.04.1999 US 132748 P; 06.05.1999 DK 61399; 01.10.1999 US 157384 P; 01.10.1999 DK 140299
(62) Divisional of application: 00922472.6
(71) Applicant: NOVO NORDISK A/S, 2880 Bagsvaerd (DK)
(72) Inventor: Flodgaard, Hans, Jakob, 2900 Hellerup (DK); Lindbom, Lennart, 75645 Uppsala (SE); Bjoern, Soeren, 2800 Lyngby (DK)
(74) Representative: Nilsson, Karin Norvin

(57) **Abstract**

The invention is related to a method for treating or preventing a disorder resulting from the release of bradykinin in said mammal, particularly a mammal which produces a HBP which binds to a HBP antagonist, e.g. a monoclonal antibody that binds to at least one epitope of human HBP, comprising administering to said mammal a HBP antagonist in an amount effective to decrease the release of bradykinin in a mammal. Furthermore, the invention is directed to methods and kits for determining if a mammal produces HBP that binds to a HBP antagonist, e.g., a monoclonal antibody that binds to at least one epitope of human HBP and a method for detecting an antagonist of HBP.

## Description

### FIELD OF THE INVENTION

The invention is related to a method for treating or preventing a disorder resulting from the release of bradykinin in a mammal, particularly a mammal which produces a heparin-binding protein which heparin-binding protein binds to a heparin-binding protein antagonist comprising administering to said mammal a heparin-binding protein antagonist in an amount effective to decrease the release of bradykinin in a mammal. Furthermore, the invention is directed to methods and kits for determining if a mammal produces heparin-binding protein that binds to a heparin-binding protein antagonist and a method for detecting an antagonist of heparin-binding protein.

### BACKGROUND OF THE INVENTION

### Inflammation

An acute inflammatory response consists of several features, including changes in vascular caliber and tone, as well as an increase in vascular permeability that results in the formation of a protein-rich exudate (Lewis in Mediators of Inflammation, Wright, Bristol, U.K., 1986).
As soon as neutrophils (PMNs) receive chemotactic signals, they marginate and adhere to the vascular endothelial cells via specific adhesion molecules synthesized on both the endothelial and neutrophil surfaces. After neutrophils are attached onto endothelial cells, there is an opening of the endothelial gaps that induce vascular permeability and permit the migration of the neutrophils into the interstitial tissue space.

The contact-phase system encompasses three enzymatic factors, factor XI (F XI), factor XII (F XII) and plasma prokallekrein (pre-kallekrein) (PK) and the nonenzymatic cofactor, H-kininogen (HK) , which forms equimolar complexes with F XI and PK, respectively. The contact phase is present on monocytes, fibroblasts and neutrophils. Specific binding sites exposed by endothelial cell and noncellular negatively charged surfaces such as kaolin, collectively referred to as the "contact phase", allow the local assembly of the critical components. The conversion of the zymogen, F XII to active enzyme, F XIIa, activates the contact-phase system itself (Colman et al., 1986, Crit. Rev. Oncol. Hematol. 5: 57-85). Reciprocal activation of surface-bound F XII and PK anchored to the surface via HK generates F XIIa and PKa thereby amplifying the initial signal. Factor XIIa can then activate Factor XI and the intrinsic pathway of coagulation is initiated. It is known that PK also hydrolyzes HK to produce the potent nonapeptide, bradykinin (Kaplan and Silverberg, 1987, Blood 70:1-15). Kinins are considered to be the primary mediators of inflammatory process that produce pain, induce vasodilation and increase vascular permeability due to a direct effect on endothelial cells, causing them to retract and permit both the migration of neutrophils and transudation of plasma constituents (Oyvin et al., 1970, Experentia 26:843-844). Local production of prostaglandins and nitric oxide, NO may also be involved (Hall, 1992, Pharmacol. Ther. 56:131-190). Therefore, activation of the contact-phase system may result in a number of deleterious effects, e.g., inflammation, septic shock, adult respiratory distress syndrome, disseminated intravascular coagulation, postoperative bleeding from cardiovascular surgery.

Specifically, the contact phase system may be activated when neutrophils bind to endothelial cells, by the presence of endotoxins and by bacterial infection (reviewed in Colman et al., 1997, Blood 90:3819-3843). For example, it has been found that in sepsis, activation of factor XII and prekallikrein result in cleavages that activate them to enzymes that rapidly react with C1-inhibitor to form factor XIIa-C1-inhibitor and kallikrein-C1-inhibitor complexes. A significant increase in kallikrein-C1-inhibitor complex formation is observed in simulated cardiopulmonary bypass.

It is found that aprotinin, an inhibitor of both plasmin and plasma kallikrein, reduces blood loss after cardiac operations and decreases the elevated postoperative bleeding time. Specifically, aprotinin is found to decrease both kallikrein-C1-inhibitor and C1-C1-inhibitor complexes in a simulated extracorporeal bypass model (Wachtfogel et al., 1993, J. Thorac. Cardiovasc Surg. 106:1). When aprotinin is added to cardiopulmonary bypass circuits that are perfused with whole blood anticoagulated with heparin, aprotinin actually complements the action of heparin (Bannan et al., 1998, Brit. J. Haem. 101:455-461). Therefore, aprotinin has an additional haemostatic beneficial effects to those found with heparin-bonded circuits.

Aprotinin also increases endothelial cell viability in hypoxic cold storage conditions when such cells are stored in organ preservation solutions and improves lung and myocardial preservation in whole organ models (Sunamori et al., 1991, Ann. Thor. Surg. 52:971-978 and Roberts et al., 1998, Ann. Thorac. Surg. 66:225-230). Furthermore, while bradykinin is found to increase vascular permeability, aprotinin decreased this vascular permeability and neutrophil count (O'Brien et al., 1997, Can. J. Physiol. Pharmacol. 75:741-749 and Dwenger et al., 1995, Eur. J. Clin. Chem. Clin. Biochem. 34:207-214).

### Heparin-Binding Protein

The covalent structure of two closely related proteins isolated from peripheral neutrophil leukocytes of human and porcine origin have recently been determined (cf. H. Flodgaard et al., 1991, Eur. J. Biochem. 197: 535-547; J. Pohl et al., 1990, FEBS Lett. 272: 200 ff.). Both proteins show a high similarity to neutrophil elastase, but owing to selective mutations of the active serine 195 and histidine 57 (chymotrypsin numbering (B.S. Hartley, "Homologies in Serine Proteinases", Phil. Trans. Roy. Soc. Series 257, 1970, p. 77 ff.)) the proteins lack protease activity. The proteins have been named human heparin-binding protein (hHBP) and porcine heparin-binding protein (pHBP), respectively, owing to their high affinity for heparin.

Schafer et al., (Schafer et al., 1984, Infect. Immun. 53:651) have named the protein cationic antimicrobial protein (CAP37) due to its antimicrobial activity. HBP binds strongly to the lipid A component of LPS and endotoxin (*K*ₐₛₛ = 0.8 x 10⁹ M⁻¹). It has been suggested that the bactericidal effect of HBP is due to binding of lipid A (Petersen et al., 1993, Eur. J. Biochem. B214: 271-279., Flodgaard et al., 1994, J. Cell. Biochem. Suppl. 18A: Abstr. E505; Pereira et al., 1993, Proc. Natl. Acad. Sci. USA 90: 4733-4737). The putative lipid A/LPS binding site of native HBP is located as an uncharged patch between a basic and an acidic patch on HBP, and comprises residues 20-26 and 41-43. In the lipid A/LPS binding site, Phe25, Cys26, Cys42, and Phe43 form a hydrophobic pocket suitable for binding of either the fatty acid chains or a glycosaminyl sugar ring of lipid A. Next to this pocket, an ionic and hydrophilic pocket (Asn20, Gln21, and Arg23) is situated, which would be well suited for binding of a glycosaminyl linked phosphate group of lipid A (Iversen et al., 1997, Nature Struct. Biology 4: 265-268).

Furthermore, in animal models of fecal peritonitis, HBP treatment has been shown to rescue mice from an otherwise lethal injury (Mercer-Jones et al., 1996, In Surgical Forum, pp. 105-108 and Wickel et al., 1997, In 4^{th} International Congress on the Immune Consequences of Trauma, Shock and Sepsis, Munich, Germany, pp. 413-416). It has been proposed that heparin-binding protein or LPS-binding portions thereof may be used to treat septic shock (WO95/28949, U.S. Pat. Nos. 5,458,874, 5,607,916 and 5,650,392).

HBP was originally studied because of its antibiotic and LPS binding properties (Gabay et al., 1989, Proc. Natl. Acad. Sci. U.S.A. 86:5610-5614 and Pereira et al., 1993, Proc. Natl. Acad. Sci. USA 90: 4733-7). However, accumulating evidence now supports the concept that HBP, in addition to its bactericidal role, is involved in the progression of inflammation due to its effect on the recruitment and activation of monocytes (Pereira et al., 1990, J. Clin. Invest. 85:1468-1476 and Rasmussen et al., 1996, FEBS Lett. 390:109-112), recruitment of T cells (Chertov et al., 1996, J. Biol. Chem. 271: 2935-2940).

HBP has been found to induce contraction in endothelial cells and fibroblasts (Østergaard and Flodgaard, 1992, J. Leuk. Biol. 51: 316-323). In relation, WO 93/05396 discloses a method for screening for HBP inhibitors by incubating HBP or a cell producing HBP with a substance suspected of being an HBP inhibitor and with tissue or cells that are capable of interacting with HBP; decreased interaction (e.g. endothelial cell contraction, indicates that the substance is an HBP inhibitor.

Application WO99/26647 discloses the use of heparin-binding protein for the modulation or prophylaxis of apoptosis of mammalian cells. This application also discloses that HBP rescues rat insulinoma cells from IL-1 induced apoptosis.

Human heparin-binding protein but not porcine heparin-binding protein has also been found to bind to aprotinin (BPTI) (Petersen et al., Eur. J. Biochem. 214:271-279). Specifically, BPTI is still capable of binding to HBP with *K*_{d} = 0.1x10⁻⁶ M (Petersen et al., 1993, Eur. J. Biochem. *B214*: 271-279). The P1 specificity of HBP has been determined to be primarily Lys or Leu (Kiczak et al., 1999, Biol. Chem. 380: 101-105). The most prominent residues in HBP which influences the binding of BPTI have been suggested to be GIy169, Gly175, Ser192, and Asp201, corresponding to Asp189, Ser195, Gly216, and Asp226 in trypsin (Petersen et al., 1993, Eur. J. Biochem. B214: 271-279). Kiczak et al., 1999, Biol. Chem. 380:101-106 constructed a library of mutants in the P1 side chain of aprotinin using phage display methods. They found that HBP shows a strong affinity for P1 Lys as well as for the uncharged P1 amino acids, Leu, Thr, Met, Gln.

### SUMMARY OF THE INVENTION

It has surprisingly been found that heparin-binding protein (HBP) serves as a signaling link in neutrophil-induced vascular leakage and activation of the contact phase system with con-commitant formation of bradykinin and that it specifically plays a role in the PK mediated cleavage of HK to obtain the bradykinin sequence. Additionally, it has been found that antagonists of HBP decrease the permeability of endothelial cells. As defined herein an "HBP antagonist" is a substance that binds to heparin-binding protein and inhibits the action of heparin-binding protein.

The invention is directed to a method for treating or preventing disorders resulting from the release of bradykinin in a mammal, particularly a human patient, particularly a mammal that produces HBP that binds to an HBP antagonist, comprising administering to said mammal in need thereof, a mammalian heparin-binding protein antagonist in an amount effective to modulate or decrease release of bradykinin in said mammal. Such disorders include but are not limited to systemic inflammatory response syndrome, ischemia reperfusion, anaphylaxis and allograft rejection. Such disorders may also include adult respiratory distress syndrome a side effect of systemic inflammatory response syndrome. Anaphylaxis may occur from inappropriate activation of PMNs during cardiopulmonary bypass, lung surgery, head trauma and major whole body trauma. The modulation or decrease in release of bradykinin is obtained by preventing contact of HBP with endothelial cells and/or with the contact-phase system. In a specific embodiment, the HBP antagonist modulates or decreases the kallikrein mediated cleavage of H-kininogen to obtain the bradykinin sequence. The invention is further directed to the use of an HBP antagonist for the preparation of a medicament useful for the treatment of systemic inflammatory response syndrome, ischemia reperfusion, anaphylaxis and allograft rejection in a patient having a HBP that binds to a HBP antagonist. HBP antagonists may further be used to treat or for the preparation of a medicament useful for the treatment of a complication of systemic inflammatory response syndrome, adult respiratory distress syndrome.

In a specific embodiment, the invention is directed to a method for preventing disorders resulting from the release of bradykinin in a mammal, particularly a human patient, particularly a mammal that produces HBP that binds to an HBP antagonist, comprising administering to said mammal in need thereof a Kunitz-type serine protease inhibitor domain or analog or derivative thereof that binds to HBP, in an amount effective to modulate or decrease release of bradykinin in said mammal.

In another specific embodiment, the invention is directed to method for treating or preventing disorders resulting from the release of bradykinin in a mammal, particularly a human patient, particularly a mammal that produces HBP that binds to a monoclonal antibody that binds to at least one epitope on HBP, wherein said epitope binds to prekallikrein-H-kininogen complex and activates release of bradykinin, comprising administering to said mammal in need thereof a monoclonal antibody that binds to at least one epitope on HBP, wherein said epitope binds to prekallikrein-H-kininogen complex and activates release of bradykinin, in an amount effective to modulate or decrease release of bradykinin in said mammal.

The invention is further directed to methods for detecting an antagonist of HBP. In one embodiment, said method comprises: (a) culturing endothelial cells in the presence of HBP and in the presence and absence of a substance suspected of being said antagonist and (b) detecting any effect of said substance on permeability of endothelial cells, wherein decreased permeability of said endothelial cells as compared to permeability of said cells when incubated in the presence of HBP without said substance indicates that said substance is an antagonist. In another embodiment, said method comprises (a) incubating HBP with a first substance that interacts with HBP and a second substance suspected of being a HBP antagonist and (b) detecting any effect of said second substance suspected of being an antagonist on interaction of HBP with said first substance.

In a specific embodiment, the invention is directed to methods for identifying a monoclonal antibody that binds to a least one epitope of HBP, particularly human HBP, wherein said epitope binds to prekallikrein-H-kininogen complex and activates release of bradykinin. In one embodiment, the method comprises: (a) culturing endothelial cells in the presence of HBP and in the presence and absence of a monoclonal antibody suspected of binding to at least one epitope of HBP, particularly human HBP, wherein said epitope binds to prekallikrein-H-kininogen complex and activates release of bradykinin, and (b) detecting any effect of said substance on permeability of endothelial cells, wherein decreased permeability of said endothelial cells as compared to permeability of said cells when incubated in the presence of HBP without said antibody indicates that said antibody is a monoclonal antibody that binds to at least one epitope on HBP, wherein said epitope binds to prekallikrein-H-kininogen complex and activates release of bradykinin. In another embodiment, the method comprises (a) incubating a prekallikrein-H-kininogen complex in the presence of HBP and in the presence and absence of a monoclonal antibody suspected of binding to at least one epitope on HBP, particularly human HBP, wherein said epitope binds to prekallikrein-H-kininogen complex and activates release of bradykinin and (b) detecting any effect of said antibody on release of bradykinin, decreased bradykinin release indicating that said antibody binds to at least said epitope on HBP.

The invention is also directed to methods and kits for determining if a mammal produces HBP that binds to a HBP antagonist. The method comprises the following steps: (a) isolating HBP or cells or tissue producing HBP from a mammal, particularly a human patient; (b) incubating said HBP or cells or tissue producing HBP with a substance, tissue, cells or component thereof which interacts with HBP and said HBP antagonist and (c ) detecting an effect of said heparin-binding antagonist on the interaction of HBP with said substance, tissue, cells or components thereof, decreased interaction indicating that said HBP binds to said HBP antagonist. The test kit comprises (a) HBP antagonist; (b) native HBP and (c) a substance, tissue, cells or component thereof which interact with HBP.

In a specific embodiment, the invention is also directed to methods and kits for determining if a mammal, particularly a human patient, produces HBP that binds to a monoclonal antibody that binds to at least on epitope on native HBP, particularly, human HBP, wherein said epitope binds to prekallikrein-H-kininogen complex and activates release of bradykinin, comprising (a) isolating HBP or cells or tissue producing HBP from said mammal; (b) culturing said HBP or cells or tissue producing HBP with endothelial cells in the presence or absence of said antibody and (c) detecting any effect of said antibody on permeability of endothelial cells, decreased permeability indicating that said HBP binds to said antibody. In another embodiment, the method comprises (a) isolating HBP or cells or tissue producing HBP from said mammal; (b) incubating said HBP or cells or tissue producing HBP with prekallikrein-kininogen complex in the presence and absence of said antibody and (c) detecting any effect of said HBP on the release of bradykinin from the prekallikrein-H-kininogen complex, decreased release of bradykinin indicating that said HBP binds to said antibody. The kit of the present invention comprises (a) a monoclonal antibody that binds to at least on epitope on native HBP, particularly, human HBP, wherein said epitope binds to prekallikrein-H-kininogen complex and activates release of bradykinin, (b) native human HBP and (c) a prekallikrein-H-kininogen complex attached to a solid support.

### BRIEF DESCRIPTION OF THE DRAWINGS

Figure 1 shows the effect of CD18 crosslinking with secondary goat anti-mouse F(ab)₂.
Figure 2 shows the dose-dependent effect of HBP (25-75 µg/ml) on permeability of endothelial cell monolayers.
Figure 3 shows the inhibition of HBP-induced increase in EC permeability with polyclonal antiserum to HBP.
Figure 4 shows the effect of anti-HBP antibodies on the actions of PMN secretions on endothelial barrier function.
Figure 5 shows inhibition of bradykinin- and HBP-induced increase in EC permeability with monoclonal antibody MBK3 to bradykinin. Bradykinin (100 nM; Figure 5a) or HBP (75 µg/ml;
Figure 5b) is administered at time zero to the lamina side of EC monolayers pre-incubated with mAb MBK3 (40 µg/ml).
Figure 6 shows inhibition of HBP-induced increase in EC permeability with monoclonal antibody PKH4 to plasma kallikrein. HBP (75 µg/ml; filled symbols) or bradykinin (100 nM; open symbols).
Figure 7 shows inhibition of HBP-induced increase in EC permeability by peptide HKH20-treatment.
Figure 8 shows inhibition of HBP-induced increase in EC permeability by peptide SDD31-treatment.
Figure 9 shows inhibition of HBP-induced increase in EC permeability by aprotinin-treatment.
Figure 10: II-6 release from human monocytes. The monocytes are cultured in 1 ml serum-free medium for 24 hours in the presence of LPS and/or native HBP / [R23S,F25E]HBP /[G175Q]HBP in the amounts indicated in the Figure.
Figure 11: Saturation of native HBP, [R23S,F25E]HBP, and [G175Q]HBP with (A) ³H-LPS and (B) Competition for ¹²⁵I-BPTI binding with fixed, increasing concentrations of unlabeled BPTI. (C) Concentrations of ¹²⁵I-BPTI shown in % 0 nM of unlabeled BPTI. The apparent difference in binding between HBP and [R23S,F25E]HBP is discussed in the "Results" section. Bars indicate standard deviations.

### DETAILED DESCRIPTION OF THE INVENTION

### Heparin-Binding Protein

In the present context, the term "heparin-binding protein" ("HBP"), refers to a protein that is (i) proteolytically inactive; (ii) stored in the azurophil granules of polymorphonuclear leukocytes; and (iii) a chemoattractant for monocytes and/or activates monocytes. The HBP may suitably be of mammalian, in particular human origin. In particular, the HBP is a mature human HBP which has at least about an 80% identity with the amino acid sequence set forth in SEQ ID NO:1, more preferably at least about 90%, even more preferably at least about 95%, and most preferably at least about 97% (hereinafter "homologous polypeptides"), which qualitative retain the activity of said HBP.

SEQ ID NO:1:
Ile Val Gly Gly Arg Lys Ala Arg Pro Arg Gln Phe Pro Phe Leu Ala Ser Ile Gln Asn Gln Gly Arg
His Phe Cys Gly Gly Ala Leu Ile His Ala Arg Phe Val Met Thr Ala Ala Ser Cys Phe Gln Ser
Gln Asn Pro Gly Val Ser Thr Val Val Leu Gly Ala Tyr Asp Leu Arg Arg Arg Glu Arg Gln Ser
Arg Gln Thr Phe Ser Ile Ser Ser Met Ser Glu Asn Gly Tyr Asp Pro Gln Gln Asn Leu Asn Asp
Leu Met Leu Leu Gln Leu Asp Arg Glu Ala Asn Leu Thr Ser Ser Val Thr Ile Leu Pro Leu Pro
Leu Gln Asn Ala Thr Val Glu Ala Gly Thr Arg Cys Gln Val Ala Gly Trp Gly Ser Gln Arg Ser
Gly Gly Arg Leu Ser Arg Phe Pro Arg Phe Val Asn Val Thr Val Thr Pro Glu Asp Gln Cys Arg
Pro Asn Asn Val Cys Thr Gly Val Leu Thr Arg Arg Gly Gly Ile Cys Asn Gly Asp Gly Gly Thr
Pro Leu Val Cys Glu Gly Leu Ala His Gly Val Ala Ser Phe Ser Leu Gly Pro Cys Gly Arg Gly
Pro Asp Phe Phe Thr Arg Val Ala Leu Phe Arg Asp Trp Ile Asp Gly Val Leu Asn Asn Pro Gly
Pro Gly Pro Ala

The amino acid sequences of the homologous polypeptides differ from the amino acid sequence set forth in SEQ ID NO: 1 by an insertion or deletion of one or more amino acid residues and/or the substitution of one or more amino acid residues by different amino acid residues. Preferably, amino acid changes are of a minor nature, that is, conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (such as arginine, lysine and histidine), acidic amino acids (such as glutamic acid and aspartic acid), polar amino acids (such as glutamine and asparagine), hydrophobic amino acids (such as leucine, isoleucine and valine), aromatic amino acids (such as phenylalanine, tryptophan and tyrosine) and small amino acids (such as glycine, alanine, serine, threonine and methionine). Amino acid substitutions which do not generally alter the specific activity are known in the art and are described, e.g., by H. Neurath and R.L. Hill, 1979, in, The Proteins, Academic Press, New York. The most commonly occurring exchanges are: Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly as well as these in reverse.

The term is specifically intended to include peptide fragments of HBP, in particular fragments with a similar chemotactic effect as native HBP. Furthermore, the HBPs used in the methods of the present invention bind to HBP antagonists such as aprotinin and/or a monoclonal antibody generated against native human HBP. Native human HBP in its mature form has the amino acid sequence shown in SEQ ID NO:1 and additionally is (i) proteolytically inactive; (ii) stored in the azurophil granules of polymorphonuclear leukocytes; and (iii) a chemoattractant for monocytes and/or activates monocytes.

HBP can be isolated from blood platelets, obtained from human blood using the procedures described in U.S. Patent No. 5,814,602. More particularly, the protein is produced by fractionation of a blood platelet extract. Column chromatography using heparin-Sepharose is expedient for this purpose. Such a chromatographic method comprising gradient elution with NaCl from 0.5 M up to 3 M of a column through which an extract of the blood platelets has been poured first, results in elution of two peaks. The first peak around 1.2 M NaCl can be measured at 280 nm as a large protein peak and is a platelet factor (PF₄) known per se. Around 1.8 M NaCl, the protein amount is below the detection limit in the system used, but the fractions in this area have angiogenic activity. The active fractions are purified additionally by microbore reverse phase HPLC on C₄ column, and at 214 nm a completely pure protein peak can be detected, and this protein is identical with the present HBP's of either porcine or human type depending on the kind of platelets used.

If the HBP is to be used to detect HBP antagonists, it should preferably be obtained via recombinant DNA methods as follows. A nucleic acid sequence encoding HBP may be prepared synthetically by established standard methods, e.g., the phosphoamidite method described by S.L. Beaucage and M.H. Caruthers, 1981, Tetrahedron Letters 22: 1859-1869, or the method described by Matthes et al., 1984, EMBO Journal 3: 801-805. According to the phosphoamidite method, oligonucleotides are synthesized, e.g., in an automatic DNA synthesizer, purified, annealed, ligated and cloned in suitable vectors.

The techniques used to isolate or clone a nucleic acid sequence encoding the heparin binding protein used in the method of the present invention are known in the art and include isolation from genomic DNA, preparation from cDNA, or a combination thereof. The cloning of the nucleic acid sequences of the present invention from such genomic DNA can be effected, e.g., by using the well known polymerase chain reaction (PCR) or antibody screening of expression libraries to detect cloned DNA fragments with shared structural features. See, e.g., Innis et al., 1990, A Guide to Methods and Application, Academic Press, New York. Other nucleic acid amplification procedures such as ligase chain reaction (LCR), ligated activated transcription (LAT) and nucleic acid sequence-based amplification (NASBA) may be used.

The nucleic acid sequence is then inserted into a recombinant expression vector that may be any vector that may conveniently be subjected to recombinant DNA procedures. The choice of vector will often depend on the host cell into which it is to be introduced. Thus, the vector may be an autonomously replicating vector, i.e., a vector which exists as an extrachromosomal entity, the replication of which is independent of chromosomal replication, e.g., a plasmid. Alternatively, the vector may be one which, when introduced into a host cell, is integrated into the host cell genome and replicated together with the chromosome(s) into which it has been integrated.

The nucleic acid sequence encoding SEQ ID NOS: 1, SEQ ID NO:2, may be operably linked to a nucleic acid encoding a heterologous pro and/or a signal sequence. Alternatively, the nucleic acid encoding SEQ ID NOS:4 (signal sequence + mature HBP) and 6 (signal sequence + pro sequence + mature HBP), SEQ ID NOS:3 and 5 respectively, may be inserted into a recombinant vector.

SEQ ID NO:2:
atcgttggcg gccggaaggc gaggccccgc cagttcccgt tcctggcctc cattcagaat caaggcaggc acttctgcgg
gggtgccctg atccatgccc gcttcgtgat gaccgcggcc agctgcttcc aaagccagaa ccccggggtt agcaccgtgg
tgctgggtgc ctatgacctg aggcggcggg agaggcagtc ccgccagacg ttttccatca gcagcatgag cga-
gaatggc tacgaccccc agcagaacct gaacgacctg atgctgcttc agctggaccg tgaggccaac
ctcaccagca gcgtgacgat actgccactg cctctgcaga acgccacggt ggaagccggc accagatgcc
aggtggccgg ctgggggagc cagcgcagtg gggggcgtct ctcccgtttt cccaggttcg tcaacgtgac tgtgaccccc
gaggaccagt gtcgccccaa caacgtgtgc accggtgtgc tcacccgccg cggtggcatc tgcaatgggg ac-
gggggcac ccccctcgtc tgcgagggcc tggcccacgg cgtggcctcc ttttccctgg ggccctgtgg ccgaggccct
 gacttcttca cccgagtggc gctcttccga gactggatcg atggcgtttt aaacaatccg  ggaccggggc cagcctag

SEQ ID NO:3:
ggctccagcc cccttttgga catcgttggc ggccggaagg cgaggccccg ccagttcccg tcctggcct ccattcagaa
tcaaggcagg cacttctgcg ggggtgccct gatccatgcc cgcttcgtga tgaccgcggc cagctgcttc caaagccaga
accccggggt tagcaccgtg gtgctgggtg cctatgacct gaggcggcgg gagaggcagt cccgccagac gttttccatc
agcagcatga gcgagaatgg ctacgacccc cagcagaacc tgaacgacct gatgctgctt
cagctggacc gtgaggccaa cctcaccagc agcgtgacga tactgccact gcctctgcag aacgccacgg
tggaagccgg caccagatgc caggtggccg gctgggggag ccagcgcagt ggggggcgtc tctcccgttt
tcccaggttc gtcaacgtga ctgtgacccc cgaggaccag tgtcgcccca acaacgtgtg caccggtgtg ctcacccgcc
gcggtggcat ctgcaatggg gacgggggca cccccctcgt ctgcgagggc ctggcccacg gcgtggcctc cttttccctg
gggccctgtg gccgaggccc tgacttcttc acccgagtgg cgctcttccg agactggatc gatggcgttt taaacaatcc
gggaccgggg ccagcctag

SEQ ID NO:4
Gly Ser Ser Pro Leu Leu Asp Ile Val Gly Gly Arg Lys Ala Arg Pro Arg Gln Phe Pro Phe Leu
Ala Ser Ile Gln Asn Gln Gly Arg His Phe Cys Gly Gly Ala Leu Ile His Ala Arg Phe Val Met Thr
Ala Ala Ser Cys Phe Gln Ser Gln Asn Pro Gly Val Ser Thr Val Val Leu Gly Ala Tyr Asp Leu
Arg Arg Arg Glu Arg Gln Ser Arg Gln Thr Phe Ser Ile Ser Ser Met Ser Glu Asn Gly Tyr Asp

Pro Gln Gln Asn Leu Asn Asp Leu Met Leu Leu Gln Leu Asp Arg Glu Ala Asn Leu Thr Ser
Ser Val Thr Ile Leu Pro Leu Pro Leu Gln Asn Ala Thr Val Glu Ala Gly Thr Arg Cys Gln Val Ala
Gly Trp Gly Ser Gln Arg Ser Gly Gly Arg Leu Ser Arg Phe Pro Arg Phe Val Asn Val Thr Val
Thr Pro Glu Asp Gln Cys Arg Pro Asn Asn Val Cys Thr Gly Val Leu Thr Arg Arg Gly Gly Ile
Cys Asn Gly Asp Gly Gly Thr Pro Leu Val Cys Glu Gly Leu Ala His Gly Val Ala Ser Phe Ser
Leu Gly Pro Cys Gly Arg Gly Pro Asp Phe Phe Thr Arg Val Ala Leu Phe Arg Asp Trp Ile Asp
Gly Val Leu Asn Asn Pro Gly Pro Gly Pro Ala

SEQ ID NO:5
atgacccggc tgacagtcct ggccctgctg gctggtctgc tggcgtcctc gagggccggc tccagccccc ttttggacat
cgttggcggc cggaaggcga ggccccgcca gttcccgttc ctggcctcca ttcagaatca aggcaggcac ttctgcgggg
gtgccctgat ccatgcccgc ttcgtgatga ccgcggccag ctgcttccaa agccagaacc ccggggttag caccgtggtg
ctgggtgcct atgacctgag gcggcgggag aggcagtccc gccagacgtt ttccatcagc agcatgagcg
agaatggcta cgacccccag cagaacctga acgacctgat gctgcttcag ctggaccgtg aggccaacct caccag-
cagc gtgacgatac tgccactgcc tctgcagaac gccacggtgg aagccggcac cagatgccag gtggccggct
gggggagcca gcgcagtggg gggcgtctct cccgttttcc caggttcgtc aacgtgactg tgacccccga ggaccagtgt
cgccccaaca acgtgtgcac cggtgtgctc acccgccgcg gtggcatctg caatggggac gggggcaccc
ccctcgtctg cgagggcctg gcccacggcg tggcctcctt ttccctgggg ccctgtggcc gaggccctga cttcttcacc
cgagtggcgc tcttccgaga ctggatcgat ggcgttttaa acaatccggg accggggcca gcctag

SEQ ID NO:6

Met Thr Arg Leu Thr Val Leu Ala Leu Leu Ala Gly Leu Leu Ala Ser  Ser Arg Ala Gly Ser Ser
Pro Leu Leu Asp Ile Val Gly Gly Arg Lys Ala Arg Pro Arg Gln Phe Pro Phe Leu Ala Ser Ile
Gln Asn Gln Gly Arg His Phe Cys Gly Gly Ala Leu Ile His Ala Arg Phe Val Met Thr Ala Ala
Ser Cys Phe Gln Ser Gln Asn Pro Gly Val Ser Thr Val Val Leu Gly Ala Tyr Asp Leu Arg Arg
Arg Glu Arg Gln Ser Arg Gln Thr Phe Ser Ile Ser Ser Met Ser Glu Asn GlyTyr Asp Pro Gln
Gln Asn Leu Asn Asp Leu Met Leu Leu Gln Leu Asp Arg Glu Ala Asn Leu Thr Ser Ser Val
Thr Ile Leu Pro Leu Pro Leu Gln Asn Ala Thr Val Glu Ala Gly Thr Arg Cys Gln Val Ala Gly
TrpGly Ser Gln Arg Ser Gly Gly Arg Leu Ser Arg Phe Pro Arg Phe Val Asn Val Thr Val Thr
Pro Glu Asp Gln Cys Arg Pro Asn Asn Val Cys Thr Gly Val Leu Thr Arg Arg Gly Gly Ile Cys
Asn Gly Asp Gly Gly Thr Pro Leu Val Cys Glu Gly Leu Ala His Gly Val Ala Ser Phe Ser Leu
Gly Pro Cys Gly Arg Gly Pro Asp Phe Phe Thr Arg Val Ala Leu Phe Arg Asp Trp Ile Asp Gly
Val Leu Asn Asn Pro Gly Pro Gly Pro Ala

The nucleic acid sequence encoding HBP may also be operably connected to a suitable terminator, such as the human growth hormone terminator (Palmiter et al., op. cit.)
The vector may further comprise other elements. These include polyadenylation signals (e.g. from SV 40 or the adenovirus 5 Elb region), transcriptional enhancer sequences (e.g. the SV 40 enhancer) and translational enhancer sequences (e.g. the ones encoding adenovirus VA RNAs). The recombinant expression vector may also comprise a DNA sequence enabling the vector to replicate in the host cell in question. The vector may also comprise a selectable marker, e.g., a gene the product of which complements a defect in the host cell, such as the gene coding for dihydrofolate reductase (DHFR) or one which confers resistance to a drug, e.g., neomycin, geneticin, ampicillin, or hygromycin.

In a specific embodiment, HBP may be produced by culturing host cells containing a DNA sequence encoding mature HBP preceded by an N-terminal extension are in a suitable culture medium under conditions permitting expression of HBP, and recovering the resulting HBP from the culture medium as N-terminally extended HBP.

The N-terminal extension may be a sequence of from about 5 to about 25 amino acid residues, in particular from about 8 to about 15 amino acid residues. The nature of the amino acid residues in the N-terminal sequence is not believed to be critical.

In order to facilitate production of mature HBP, it is generally preferred that the DNA sequence encoding N-terminally extended HBP includes a DNA sequence encoding a protease cleavage site located between the DNA sequence encoding the N-terminal extension and the DNA sequence encoding mature HBP. Examples of suitable protease cleavage sites are an enterokinase cleavage site with the amino acid sequence, a Factor Xa cleavage site with the amino acid sequence.

Alternatively, host cells containing a DNA encoding mature HBP preceded by a signal sequence may be cultured in a suitable culture medium under conditions permitting expression of HBP and the resulting HBP is recovered from the culture medium as mature HBP.

The procedures used to ligate the nucleic acid sequences coding for HBP or the N-terminally extended HBP, Δpro-HBP (signal + mature HBP), the promoter and the terminator, respectively, and to insert them into suitable vectors containing the information necessary for replication, are well known to persons skilled in the art (cf., for instance, Sambrook et al., op.cit.).

The host cell(s) into which the expression vector is introduced may be any cell(s) which is capable of producing HBP and is preferably a eukaryotic cell(s), such as invertebrate (insect) cell(s) or vertebrate cell(s), e.g., mammalian cell(s), in particular insect and mammalian cell(s). In a preferred embodiment, the mammalian cell is a cell which can be cultured under anaerobic conditions after transfection with a nucleic acid encoding mammalian HBP. In a more preferred embodiment, the mammalian cell is an adenovirus-transformed cell(s) or derived from an embryonic cell(s). As defined herein, a cell derived from an embryonic cell is a cell(s) obtained from a primary culture of embryonic cells or a cell(s) from a cell line originally passaged from a primary culture of embryonic cells. An example of such an adenovirus-transformed cell or embryonically-derived cell is a human embryonic kidney (HEK) cell(s), particularly HEK 293 cells. The insect cell may, for example, be Lepidoptera or Drosophila cells.

Methods for transfecting mammalian cells and expressing DNA sequences introduced in the cells are described in e.g., Kaufman and Sharp, 1982, J. Mol. Biol. 159:601-621; Southern and Berg, 1982, J. Mol. Appl. Genet. 1:327-341; Loyter et al., 1982, Proc. Natl. Acad. Sci. USA 79:422-426; Wigler et al., 1978, Cell 14:725; Corsaro and Pearson, 1981, Somatic Cell Genetics 7:603, Graham and van der Eb, 1973, Virology 52:456; Fraley et al., 1980, JBC 225:10431; Capecchi, 1980, Cell 22:479; Wiberg et al., 1983, NAR 11:7287; and Neumann et al., 1982, EMBO J. 1:841-845. Insect cells may suitably be transfected with a baculovirus vector as described in U.S. Patent No. 4,745,051.

The medium used to culture the cells may be any conventional medium suitable for growing mammalian cells, such as a serum-containing or serum-free medium containing appropriate supplements, or a suitable medium for growing mammalian cells. Suitable media are available from commercial suppliers or may be prepared according to published recipes (e.g. in catalogues of the American Type Culture Collection). The cells are then screened for antibiotic resistance. Subsequently, the selected clones are subsequently assayed for HBP activity using assays known in the art such as a chemotaxis assay and assaying for cytokine release from monocytes (see, for example, Rasmussen et al., 1996, FEBS Lett. 390:109-112).

The HBP produced by the cells may then be recovered from the culture medium by conventional procedures including separating the host cells from the medium by centrifugation or filtration, precipitating the proteinaceous components of the supernatant or filtrate by means of a salt, e.g., ammonium sulphate, purification by a variety of chromatographic procedures, e.g., ion exchange chromatography, affinity chromatography, or the like.

If the HBP is N-terminally extended HBP, after recovery from the culture medium, the N-terminally extended HBP may advantageously be cleaved with a suitable protease to produce mature (and active) HBP. Examples of suitable enzymes include but are not limited to enterokinase and Factor Xa.

### HBP ANTAGONISTS

An HBP antagonist may be either anti-HBP polyclonal or monoclonal antibodies. The following procedure may be used to obtain anti-HBP polyclonal antibodies. For the production of antibody, various host animals can be immunized by injection with the HBP, preferably human HBP, including but not limited to rabbits, mice, rats, sheep, goats, etc. In one embodiment, HBP can be conjugated to an immunogenic carrier, e.g., bovine serum albumin (BSA) or keyhole limpet hemocyanin (KLH). Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and Corynebacterium parvum.

The following procedure may be used to obtain monoclonal antibodies that bind to at least one epitope of HBP, in particular, human HBP, wherein said epitope binds to prekallikrein-H-kininogen complex and activates release of bradykinin. In a preferred embodiment, the monoclonal antibody is a human monoclonal antibody. Any technique that provides for the production of antibody molecules by continuous cell lines in culture may be used. These include but are not limited to the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). In an additional embodiment of the invention, monoclonal antibodies can be produced in transgenic animals utilizing recent technology (PCT/US90/02545; Green, 1999, J. Immunol. Methods 231:11-23 and U.S. Pat. Nos. 5,625,126 and 5,633,425). According to the invention, human antibodies may be used and can be obtained by using human hybridomas (Cote et al., 1983, Proc. Natl. Acad. Sci. U.S.A. 80:2026-2030) or by transforming human B cells with EBV virus in vitro (Cole et al., 1985, in Monoclonal Antibodies and Cancer Therapy, Alan R. Liss, pp. 77-96).

According to the invention, techniques described for the production of single chain antibodies (U.S. Pat. No. 4,946,778) can be adapted to produce HBP-specific single chain antibodies. An additional embodiment of the invention utilizes the techniques described for the construction of Fab expression libraries (Huse et al., 1989, Science 246:1275-1281) to allow rapid and easy identification of monoclonal Fab fragments with the desired specificity for HBP.

Antibody fragments which contain the idiotype of the antibody molecule can be generated by known techniques. For example, such fragments include but are not limited to: the F(ab').sub.2 fragment which can be produced by pepsin digestion of the antibody molecule; the Fab' fragments which can be generated by reducing the disulfide bridges of the F(ab').sub.2, fragment, and the Fab fragments which can be generated by treating the antibody molecule with papain and a reducing agent.

In the production of antibodies, screening for the desired antibody can be accomplished by techniques known in the art, e.g., radioimmunoassay, ELISA
(enzyme-linked immunosorbent assay), "sandwich" immunoassays, immunoradiometric assays, gel diffusion precipitin reactions, immunodiffusion assays, in situ immunoassays (using colloidal gold, enzyme or radioisotope labels, for example), western blots, precipitation reactions, agglutination assays (e.g., gel agglutination assays, hemagglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

Alternatively, a library of anti-HBP antibodies can be generated using phage-display techniques (see, for example, Koscielska et al., 1998, Acta Biochimica Polinica 45:705-720).
The library may be screened and the strongest binders may be used as HBP antagonists.

The HBP antagonist may also be a polypeptide comprising a Kunitz-type serine protease inhibitor domain that binds to HBP. Peptide comprising Kunitz-type serine protease inhibitor domains generally contain about 60 residues and six specifically spaced cysteines that are present in disulfide bonds. In a specific embodiment, if the HBP is human HBP, the Kunitz-type inhibitor may be aprotinin also known as BPTI or analog thereof that binds to HBP. In a preferred embodiment, the aprotinin analog has mutations at the 12-19 and 34-39 positions, especially preferred are mutations at the 15-19 positions. Other analogs that may be used are disclosed in U.S. Patent Nos. 5,162,498, 5,316,923, 5,395,922, 5,514,585, 5,510,249, 5,591,603, 5,618,915, 5,621,074, 5,673,090, 5,618,696 and 5,576,294. In a most specific embodiment, the mutations are A16H, K15A, I18M, I19S, R17T.

Large libraries (10⁷-10⁹) of polypeptides comprising kunitz-type domains may be obtained using a variety of approaches. For example, error-prone PCR (Saiki et al., 1988, Science 293:487-491) may be applied to each individual kunitz-type domain. This procedure will randomly incorporate the incorrect nucleotide in the gene for each corresponding kunitz-type domain by running the PCR reaction under conditions where the Taq polymerase incorporates wrong nucleotides in the amplification step. The library may be displayed on phage particles to select the best binder to HBP.

Alternatively, specific loops may be subjected to mutagenesis by the use of degenerate oligonucleotides (Schier et al., 1996, Gene 169:147-153). Synthetic random oligonucleotides are incorporated into the kunitz-type domain during the PCR reaction generating a library with variants in a particular loop.

In yet another approach, libraries may be generated via DNA shuffling (Stemmer et al., 1994, Nature 370:389-391). This method can be used for recombination between homologous kunitz-type domains. In short, DNA coding for each variant is mixed and fragmented to small pieces. These pieces are then reassembled in a regular PCR reaction. During this reaction, the small pieces will join each other because they are complementary to each other leading recombination of the genes included.

### Detection Methods

Heparin-binding protein (HBP) antagonists may be detected by determining if a substance suspected of being a HBP antagonist binds to HBP by (a) incubating HBP with a first substance that interacts with HBP and a second substance suspected of being a HBP antagonist and (b) detecting any effect of said second substance suspected of being an antagonist on interaction of HBP with said first substance, wherein decreased interaction of HBP with first substance is indicative that second substance is an HBP antagonist.

The HBP or first substance may be labeled. The label is preferably selected from the group consisting of enzymes, colored or fluorescent substances, radioactive isotopes and complexing agents.

Examples of enzymes useful as labeled substances re peroxidases (such as horseradish peroxidase), phosphatases (such as acid or alkaline phosphatase), B-galactosidase, urease, glucose oxidase, carbonic anhydrase, acetylcholinersterase, glucoamylase, lysozyme, malate dehydrogenase, glucose-6-phosphate dehydrogenase, b-glucosidase, proteases, pyruvate decarboxylase, esterases, luciferase, etc.

Enzymes are not in themselves detectable but must be combined with a substrate to catalyze a reaction, the end product of which is detectable. Examples of substrates which may be employed in the method according to the invention include hydrogen peroxide/tetramethylbenzidine or chloronaphthole or o-phehylenediamine or 3-(p-hydroxyphenyl) propionic acid or luminol, indoxyl phosphate, p-nitrophenylphosphate, nitophenyl galactose, 4-methyl umbelliferyl-D-galactopyranoside or luciferin.

Alternatively, the labeled substance may comprise colored or fluorescent substances, including gold particles, colored or fluorescent latex particles, dye particles, fluorescein, phycoerythrin or phycocyanin.

Radioactive isotopes which may be used for the present purpose may be selected from ¹²⁵I, ¹³¹I, ¹¹¹In, ³H, ³²P, ¹⁴C and ³⁵S. The radioactivity emitted by these isotopes may be measured in a gamma-counter or a scintillation counter in a manner known in the art.

Complexing agents which may be employed for the present purpose may be selected from biotin (which complexes with avidin or streptavidin), avidin (which complexes with biotin), Protein A (which complexes with immunoglobulins) and lectins (complexing with carbohydrate receptors). Since the complex is not directly detectable, it is necessary to label the substance with which the complexing agent forms a complex. The labelling may be carried out with any one of the labeled substances mentioned above fro the labelling of the enzyme.

In a specific embodiment, the binding of HBP antagonist to HBP may be detected by using, for example, SPA technology from Amersham Pharmacia Biotech. Briefly, biotinylated plasma membranes from umbilical vein endothelial cells (HUVEC) are bound to streptavidin conjugated PVT SPA beads. These beads may be obtained from Amersham Pharmacia Biotech. Labeled human kininogen (for example, ³H, ¹²⁵I) is added to the membranes until the binding sites for kininogen are saturated. HBP is added in increasing concentrations to displace the labeled kininogen and the decline in incorporated label is measured. A standard displacement curve is prepared. HBP antagonist is tested for its ability to inhibit HBP mediated displacement of radioactive kininogen for the plasma membranes. Specifically, HBP is incubated with various concentrations up to molar excess of HBP antagonists and their ability to inhibit HBP mediated displacement of the labeled kininogen from the membranes is determined.

Alternatively, HBP antagonists may be detected by culturing endothelial cells in the presence of HBP and in the presence and absence of a substance suspected of being an antagonist. In a specific embodiment, the antagonist is a monoclonal antibody that binds to at least one epitope of HBP, preferably human HBP, wherein said epitope binds to prekallikrein-H-kininogen complex and activates release of bradykinin. In a preferred embodiment, the monoclonal antibody is a human monoclonal antibody. In one embodiment, the antibody and HBP are preincubated together before incubating with endothelial cells for about 10 minutes at 37ºC. The effect of the substance on the permeability of endothelial cells is determined by measuring transendothelial electrical resistance or determining albumin clearance as described in the Examples, *infra.* A decrease in permeability would indicate that the substance does act as an HBP antagonist.

In a specific embodiment, a monoclonal antibody binding to at least one epitope of HBP, wherein said epitope binds to prekallikrein-H-kininogen complex and activates release of bradykinin, can be detected by incubating the antibody with HBP and a prekallikrein-H-kininogen complex. As will be described in further detail in the Examples, HBP binds to the prekallikrein-H-kininogen complex thereby stimulating the release of bradykinin. A monoclonal antibody that binds to at least one epitope of HBP would decrease or prevent the release of bradykinin. The release of bradykinin may be assayed using procedures known in the art, e.g., immunoassay.

In one embodiment, coating buffer containing 0.05% Tween, 15.9 mM disodium carbonate 35 mM sodium hydrogen carbonate, pH 9.6. H-kininogen is added and incubated at 4ºC overnight. The coating buffer is removed and the plate is washed with Tris buffer, pH 7.4 containing about 50 M zinc. Prekallikrein is then added and the mixture is incubated for 1 hr. at room temperature. HBP in Tris buffer is subsequently added and samples are removed at about 10 minute intervals for about 1 hour beginning about 5 minutes after adding HBP and assayed for bradykinin release. In one embodiment, bradykinin release may be assayed using a commercially available ELISA assay (MARKIT-M Bradykin, Dainippon Pharmaceutical Co., Ltd.). In such an assay, bradykinin in a sample and peroxidase labeled bradykinin are allowed to react competitvely with anti-BK antibody (rabbit) captured by anti-rabbit IgG antibody (goat) coated on a microstrip well. The bradykinin concentration is determined from the enzyme activity of peroxidase labeled bradykinin bound to anti-bradykinin antibody.

Alternatively, prekallikrein and H-kininogen may be added in equimolar amounts to a monolayer of endothelial cells and incubated for 1 hr. at 37ºC. HBP is subsequently added and samples are removed at about 10 minute intervals for about 1 hour beginning about 5 minutes after adding HBP and assayed for bradykinin release using procedures described above.

In yet another embodiment, prekallikrein and H-kininogen may be added in equimolar amounts to a plasma solution containing ACE (angiotensin converting enzyme) inhibitor and incubated for 1 hr. at 37ºC. HBP is subsequently added and samples are removed at about 10 minute intervals for about 1 hour beginning about 5 minutes after adding HBP and assayed for bradykinin release using procedures described above.

### Treatment Methods

The composition of the present invention comprises part of an aqueous solution. The solution preferably is physiologically acceptable so that in addition to delivery of the desired construct to the patient, the solution does not otherwise adversely affect the patient's electrolyte and volume balance. The aqueous medium for the composition thus may comprise, for example, normal physiological saline (0.9% NaCl, 0.15M), pH 7-7.4 or other pharmaceutically acceptable salts thereof. Useful solutions may be prepared by any of the methods well known in the pharmaceutical art, described, for example, in "Remington's Pharmaceutical Sciences", (Gennaro, A., ed.), Mack Pub., 1990.

The concentration of HBP antagonist may vary widely, i.e. from less than about 0.5%, such as from 1%, to as much as 15-20% by weight. A unit dosage of the composition may typically contain from about 10 mg to about 1 g of HBP antagonist. Therapeutically effective dosages may be determined by either in vitro or in vivo methods.

HBP antagonist is administered topically, subcutaneously or by intravenous injection. Dosages will be prescribed by the physician according to the particular condition and the particular individual to be treated. Dosages and frequency is carefully adapted and adjusted according to parameters determined by the physician in charge. A preferred administration route may be, e.g., injections intraperitoneally. Intravenous intraperitoneal injections of HBP antagonist may be given per 24 hours in the range of from 0.1-100 mg, especially 0.5-50 mg, in particular 1-25 mg per kg body weight. The dose may be given 1-4 times per 24 hours or administered continuously through a catheter. Alternatively, treatments may be given via continuous infusion during surgery intravenously, then subsequent treatment for 1-4 days after surgery.

The HBP antagonist will be administered only to those patients producing HBP that binds to the HBP antagonist. To determine if the patient produces such HBP, the HBP from the patient will be isolated from the platelets from the blood of patients using procedures described above. The binding of HBP to HBP antagonist (for example, a monoclonal antibody) or effect of HBP on the effect on endothelial cell permeability may be detected using procedures described above and compared to the effects of native HBP. Alternatively, the effect of HBP on endothelial cell or fibroblast cell contraction or monocyte aggregation in the presence or absence of HBP antagonist may also be determined using the procedures described in WO93/05396. A decreased degree of cell contraction or aggregation indicates the presence of an interaction between HBP and HBP antagonist. A test kit that may be used to determine if a patient produces such HBP comprises comprising (a) HBP antagonist; (b) a HBP or cell producing HBP and (c) a substance, tissue, cells or component thereof which interact with HBP.

In a specific embodiment, HBP or cells or tissue producing HBP is isolated from a patient. The HBP is or cells or tissue producing said HBP are incubated with endothelial cells and the effect on permeability is determined in the presence and absence of monoclonal antibody that binds to at least one epitope of HBP, wherein said epitope binds to prekallikrein-H-kininogen complex and activates release of bradykinin. This effect is compared to the effect of native human HBP. In a specific embodiment, the native human HBP may be recombinant HBP which bind to such a monoclonal antibody. Alternatively, HBP or cells or tissue producing HBP are incubated in the presence of prekallikrein-H-kininogen complex and in the presence of monoclonal antibody. The effect of the monoclonal antibody on bradykinin release is determined. A decrease in bradykinin release in the presence of antibody indicates that the patient produces HBP that binds to the antibody. In such a situation, the test kit would comprise (a) a monoclonal antibody that binds to at least one epitope on HBP, wherein said epitope binds to prekallikrein-H-kininogen complex and activates release of bradykinin; (b) native human HBP and (c) prekallikrein-H-kininogen complex attached to a solid support.

### EXAMPLES

### Example 1: Activated neutrophils trigger alterations in endothelial barrier function through signaling via β2 integrins

### MATERIALS AND METHODS

### Reagents

Medium-199, RPMI-1640 (containing L-glutamine), fetal bovine serum (FBS), trypsin-EDTA, phosphate buffered saline (PBS) and Hanks balanced salt solution (HBSS) are from Life Technologies (Gaithersburg, MD, USA). Gelatin, collagenase, penicillin, streptomycin, catalase, bovine serum albumin (BSA), N-formyl-methionyl-leucyl-phenylalanine (fMLP), Evans blue dye, hexadecyltrimethylammonium bromide and tetramethylbenzidine are obtained from Sigma Chemical Co. (St. Louis, MO, USA). H₂O₂ is obtained from E. Merck (Darmstadt, Germany). Biomatrix I is obtained from Biomedical Technologies Inc. (Stoughton, MA, USA), and Dextran 70 (Macrodex) and Ficoll-Paque are from Pharmacia Biotech AB (Uppsala, Sweden). The basal culture medium consisted of a 1:1 mixture of M-199 and RPMI-1640 supplemented with 20% heat inactivated FBS, penicillin (100 U/ml) and streptomycin (100µg/ml). The monoclonal antibody IB4 against the common β-chain (CD18) of β₂-integrins is provided by Dr. Claes Lundberg, Pharmacia & Upjohn (Uppsala, Sweden) by courtesy of Dr. Samuel D. Wright, Rockefeller University, mAb 60.1 recognizing integrin αₘ chain (CD11 b) is provided by Dr. Manuel Patarroyo, Karolinska Institutet, mAb DREG 200 (anti-L-selectin) is a gift of Dr. Eugene Butcher, Stanford University, mAb SK11 (anti-L-selectin) is from Becton Dickinson (San José, CA) and mAb G44-26 (anti-CD44) is obtained from PharM-ingen (San Diego, CA). F(ab')₂ fragment of goat anti-mouse IgG is obtained from Jackson ImmunoResearch Laboratories, Inc. (West Grove, PA), FITC-conjugated F(ab')₂ fragment of rabbit anti-mouse IgG is from Dako A/S (Glostrup, Denmark).

### Endothelial cells

Human umbilical vein endothelial cells (HUVEC) and bovine aorta endothelial cells (BAEC) are isolated and cultured as previously described (Gautam *et al*. 1998, Brit. J. Pharm. 125:1109-1114). HUVEC or BAEC in the first to fifth passages are detached by brief (2 min) trypsin-EDTA treatment (0.25% trypsin/0.01 % EDTA) and replated onto either 0.2 µm pore size Anopore inorganic membrane or 3.0 µm pore size polycarbonate filters (Tissue Culture inserts, 10 mm; NUNC, Roskilde, Denmark). In order to promote cellular differentiation and enhance attachment of endothelial cells (ECs), the filters are pretreated with 50 µl Biomatrix I (167 µg/ml) and air-dried. ECs are seeded at a density of 2 x 10⁵ cells per filter and incubated in culture medium at 37°C in a humidified atmosphere of 5% CO₂ in air. The EC are grown to confluent monolayers, as assessed by daily microscopic observation and measurement of the electrical resistance across the monolayer (Gautam *et al*. 1998, Brit. J. Pharm. 125:1109-1114).

### Measurement of EC barrier function

Two different approaches are used for assessment of stimulation-induced changes in EC permeability. For measurement of transendothelial electrical resistance (TEER), the filter insert with EC is transferred to a resistance measurement chamber (for details, see Gautam *et al*. 1998, Brit. J. Pharm. 125:1109-1114). The chamber (lower/luminal compartment) with the filter insert (upper/luminal compartment), filled with 2 ml and 400µl culture medium respectively is placed in a cell culture incubator. Electrical resistance across the EC monolayer is measured at 37°C with electrodes in each compartment exactly positioned in relation to each other and to the monolayer. The electrical resistance of individual EC monolayers is obtained by subtracting the resistance of the corresponding naked filter coated with Biomatrix as measured prior to seeding of the endothelial cells.

In separate experiments, Evans blue dye-conjugated albumin (EBA) is used as a marker for macromolecular permeability of the EC monolayers. Prior to stimulation of EC, the culture medium in the upper compartment is exchanged for medium containing EBA (culture medium containing 4% BSA mixed with Evans blue dye at a final concentration of 0.67 mg/ml). The EBA concentration in fluid samples of the upper and lower compartment is determined spectrophotometrically through measurement of absorbency at 620 nm (Titertek Multiskan MCC; Flow Laboratories, Solna, Sweden). Albumin clearance is calculated according to the relationship: V1 = A2 x V2 x 1/A1, where V1 is the clearance volume (i.e. the theoretical volume of the luminal medium cleared from albumin by diffusion to the abluminal compartment), V2 is the abluminal volume, and A1 and A2 is the absorbency of the luminal and abluminal medium, respectively. The baseline clearance in absence of any stimulus, averaging 0.08 ± 0.03 µl/min, is subtracted from the clearance volume obtained in response to respective specific stimulus.

### Preparation and quantification of PMNs

Human PMNs are isolated from leukocyte-rich plasma as described (Gautam et al., 1998, Brit. J. Pharm. 125:1109-1114), and resuspended in culture medium at a concentration of 2-5 x 10⁷ cells/ml. The purity of the PMNs is > 98% and their viability by Trypan blue exclusion is >95%. Before being added to the EC monolayers, the PMNs are incubated for 30 min with monoclonal antibodies against cell surface molecules for subsequent cross-linking with secondary mAb (see experimental procedures).

In some experiments, cell surface expression of CD11b/CD18 is upregulated through temperature transition of the PMNs prior to use. PMNs in the presence of anti-CD18 mAb IB4 are incubated at 4°C for 10 min, transferred to 37°C for 10 min and then returned to 4°C for another 10 min followed by two washes. PMNs not subjected to antibody treatment are prepared according to the same protocol using vehicle instead of antibody.

PMN adhesion to EC and transendothelial migration are quantified by assaying the PMN-specific enzyme myeloperoxidase (MPO). In brief, PMNs are lysed in 0.5 % hexadecyltrimethylammonium bromide and centrifuged, and the enzyme activity in the supernatant determined spectrophotometrically as the change in absorbency at 650 nm that occurs in the redox reaction of H₂O₂-tetramethylbenzidine catalyzed by MPO (Suzuki *et al*., 1983, Anal. Biochem. 132:345-352). MPO activity of adherent and transmigrated PMNs, respectively, is related to that of the total number of PMNs added to the EC monolayer.

### Experimental procedures

Before transferring the filter insert to the resistance measurement chamber (kept at 37°C), the medium in the insert and in the chamber is replaced with fresh culture medium (37°C) supplemented with 10 mM Hepes. In some experiments, the EC monolayer is incubated before stimulation with 10 µg/ml Herbimycin A for 15 min at 37°C and washed twice. PMNs (2 x 10⁶), with or without monoclonal antibodies bound to cell surface molecules, are added to the upper compartment (PMN:EC ratio = 10:1) and allowed to sediment for 10 min onto the EC monolayer. Activation of PMN is induced either with fMLP (10⁻⁷ M) added to the lower compartment or through antibody cross-linking of cell surface molecules by secondary goat-anti-mouse IgG F(ab')₂ fragments. Transendothelial electrical resistance is measured before and every minute after start of stimulation until a plateau phase in the resistance change is reached, and then at 5-min intervals. In experiments where albumin permeability and PMN migration across the EC monolayer are studied, the medium in the insert contained EBA. After challenge, the filter insert is moved at 5-10 min intervals to new wells containing fresh medium. All incubations are performed at 37°C. At the end of the experiment, the lower wells are centrifuged at 425g and 4°C for 20 min and the supernatant is analyzed for EBA content. PMN that had migrated across the monolayer are quantified through analysis of the MPO-activity of the pellet remaining in the well. The medium in the filter insert is aspirated for quantification of non-adherent PMN, and the filter membrane with EC is removed from the insert to quantify the adherent fraction of PMN.

In separate experiments, purified PMNs, pretreated with the anti-CD18 mAb IB4 (6 µg per 2 x 10⁶ cells) for 30 min, are incubated in culture medium with or without goat-anti-mouse F(ab')₂ for 10 min at 37°C. The PMNs are sedimented by centrifugation at 300xg for 15 min at room temperature and the cell-free supernatant containing PMN secretion products is decanted for use in experiments *in vivo* (see below) or with the EC-monolayers. In this latter setting, the medium in the filter/EC insert is exchanged for the PMN secretion-containing medium, and effects on TEER and albumin permeability are recorded as described above. In some cases, the PMN secretion-containing medium is heat-incubated (80°C for 15 min) before use.

### Determination of EC intracellular [Ca²⁺] and f-actin formation

The Ca²⁺ sensitive fluorescent probe fluo-3/AM (Molecular Probes Europe BV, Leiden, The Netherlands) is used for determination of changes in EC intracellular free Ca²⁺. Confluent BAEC monolayers on filters are incubated for 30 min at 37°C with fluo-3/AM (3µM in HBSS containing 2% FCS and 10 mM HEPES) added to both the apical and basolateral surface. The monolayers are washed three times and incubated with fresh HBSS in the dark for 20 min at room temperature to allow complete hydrolysis of the dye ester. Anti-CD18 or anti-L-selectin mAb treated PMNs are added to ECs and receptor cross-linking is induced with secondary mAb as described above. In separate experiments, PMN secretion containing medium (see above) is used to stimulate ECs in the absence of PMNs. Changes in EC [Ca²⁺]ᵢ in response to these stimuli are measured through continuous registration of fluorescence intensity with a laser-scanning confocal imaging system (Insight Plus; Meridian Instruments Inc., Okemon, Michigan).

For analysis of f-actin formation in ECs in response to PMN activation, confluent EC monolayers grown on Biomatrix coated coverslips are incubated for 15 min at 37°C either with PMN subjected to CD18 antibody cross-linking or with PMN secretion containing medium identical to the procedures described above. Incubation of ECs with IB4-treated PMN served as control. The monolayers are fixed in 3.7% formaldehyde in PBS for 10 min at room temperature, washed twice and permeabilized with 0.2% Triton X-1 00 in PBS (1 min at 4° C). The cells are washed twice and stained for actin filaments with FITC-conjugated phalloidin for 20 min at 37°C. After three additional washes the endothelial cells are viewed in a laser-scanning confocal imaging system (Insight Plus; Meridian Instruments Inc., Okemon, Michigan).

### In vivo experiments

In order to study the effect of PMN secretion products on microvessel permeability *in vivo,* intravital microscopy of the hamster cheek pouch microcirculation is applied. As previously described in detail (Raud & Lindbom, 1994, Immunology of the Microcirculation, Academic Press, 1994, chapter 7), the left cheek pouch of anaesthetized Syrian golden hamsters is everted and prepared for microscopic observation under continuous superfusion with a bicarbonate buffer maintaining physiological levels of temperature, pH and gas tensions. FITC-conjugated dextran (Mw: 150.000) injected i.v. (250 mg/kg body weight) is used as plasma tracer to permit visualization of changes in vascular permeability to macromolecules. The microvasculature in the exposed cheek pouch is observed under low magnification in fluorescent light with a Leitz Orthoplan microscope and recorded on video. After control recordings, superfusion is stopped and 1 ml of medium containing CD18 cross-linking-evoked PMN secretion at 37° C is topically applied to the cheek pouch. In parallel experiments, anti-CD18-treated PMN medium is not subjected to cross-linking is used.

### RESULTS

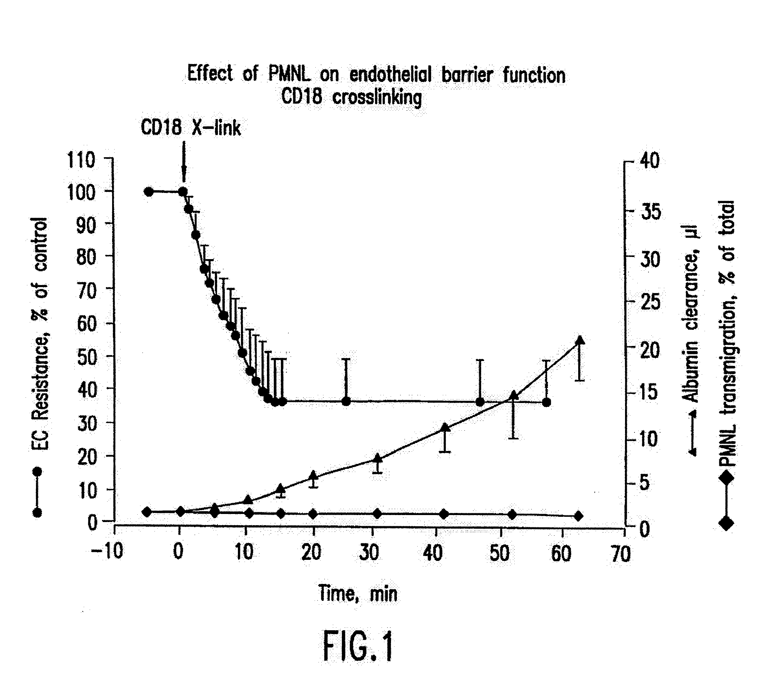

PMNs (2x10⁶), pre-treated with anti-CD18 mAb (IB4), are added to the EC/filter insert (luminal side of EC/upper compartment of the resistance chamber) and allowed to settle for 10 min. CD18 cross-linking with secondary goat anti-mouse F(ab)₂ elicited a marked decrease in TEER, which is manifested within 1 min, reached its maximum (36±13 of control) after 15 min and remained at this level throughout the observation period (Figure 1). Neither treatment of PMNs with IB4 alone nor with secondary mAb in the absence of IB4 caused any change in TEER.

The capacity of CD18 cross-linking in causing increased macromolecular flux and PMN migration across the EC monolayer is studied in additional experiments. PMNs pre-treated with saturating concentration of IB4 are added to the EC/filter insert and allowed to settle for 10 min in medium containing EBA. CD18 cross-linking is similarly induced with goat anti-mouse F(ab)₂ and the PMN/EBA containing filter inserts are moved at regular intervals to new wells containing fresh medium. In the absence of secondary antibody, there is a minute and continuous clearance of albumin, 0.08±0.03 µl·min⁻¹ (mean±SD, n=13), which did not change with time and which exactly matched the spontaneous clearance rate found in filter inserts containing untreated PMNs. By contrast, activation of the PMNs by CD18 cross-linking elicited augmented albumin clearance, which increased progressively with time (Figure 1). The net increase in albumin clearance rose from 0.5±0.1 µl during the first 5-min period to a total of 20.0±4.5 µl (mean±SD, n=10) in 60 min. Distinct from increased albumin clearance, CD18 cross-linking caused no measurable PMN adhesion to EC or transendothelial migration (Figure 1). Analysis of MPO activity in the different compartments 60 min after CD18 cross-linking revealed that 91±6 % (mean±SD, n=9) of added PMNs are recovered in medium collected from the upper compartment, whereas no significant MPO-activity is detected in the EC/filter-unit or in the medium of the lower compartment. Direct microscopic observation of transparent filters washed at the end of the experiment confirmed the absence of PMNs adherent to the EC monolayer.

Monolayers of endothelial cells are labelled with the Ca²⁺-sensitive fluorophore fluo-3, and stimulus-induced changes in EC [Ca²⁺]ᵢ monitored by confocal laser scanning microscopy. Adding anti-CD18 treated PMNs to the EC monolayer caused no change in EC [Ca²⁺]. However, CD18 cross-linking with secondary mAb elicited a rapid increase in EC cytosolic free [Ca²⁺], which peaks after approximately 100 sec and then subsided slowly. Antibody cross-linking of L-selectin in an equivalent manner failed to induce any change in EC [Ca²⁺], whereas a Ca²⁺-response similar to that induced by CD18 cross-linking is seen after stimulation of untreated PMNs with fMLP (c.f. Gautam *et al*, 1998, Brit. J. Pharmacol. 125:1109-1114). A causal link between intracellular Ca²⁺-activity changes and the functional response of EC to PMN activation is evidenced by experiments where the endothelial cells are pre-treated with the calcium-chelating agent BAPTA AM. This treatment completely inhibited both the rise in EC free intracellular Ca²⁺ and the change in TEER in response to CD18 cross-linking.

In further experiments, EC monolayers are incubated with PMNs and then stained for actin filaments with FITC-conjugated phalloidin. Confocal laser scanning microscopy revealed that endothelial cells incubated with IB4-treated PMN in absence of secondary antibody exhibited few stress fibers and a thin band of actin along cell margins in direct apposition with an adjacent cell. On the other hand, endothelial cells incubated with PMN subjected to CD18 cross-linking presented a marked increase in f-actin content and in the number and density of stress fibers.

To investigate if factor(s) secreted by the PMNs govern the response ultimately leading to increased EC permeability, PMNs (2x10⁶), pre-treated with anti-CD18 mAb IB4 and washed to remove unbound antibody, are incubated with goat anti-mouse F(ab')₂ for 30 min and then spun down by gentle centrifugation. Addition of cell-free supernatant to the EC monolayer elicited a fall in TEER and an increase in albumin clearance, both of which with the same magnitude and time course as after CD8 cross-linking in PMNs added to EC. Also, the supernatant caused changes in EC[Ca²⁺]ᵢ and in f-actin content and distribution. Moreover, topical administration of the PMN secretion-containing supernatant to the hamster cheek pouch in vivo-preparation elicited prompt leakage of plasma from postcapillary and small venules. Leaky spots are observed already within 1.5 min after application, and a maximal response manifested after approximately 5 min. After wash of the tissue with buffer the leakage slowly subsided. This effect on microvessel permeability is lost after heat-treatment of the PMN-derived secretion indicating that a heat-sensitive factor(s) is responsible for its permeability-increasing activity. Application to the cheek pouch of supernatant of IB4-treated PMNs not subjected to cross-linking did not cause any visible plasma leakage.

### DISCUSSION

Polymorphonuclear leukocyte adhesion to the endothelial lining and recruitment to extravascular tissue in acute inflammation is critically dependent on the function of β2 integrins. Increased vascular permeability for macromolecules is induced in conjunction with this cellular response leading to plasma exudation and edema formation. The results of the above-described experiments have provided evidence for a causal link between transmembrane (outside-in) signaling by β2 integrins in activated PMNs and the capacity of these cells to induce EC barrier dysfunction. It is shown that antibody-induced ligation and clustering of β2 integrins triggers an increase in EC permeability similar to that seen after chemoattractant stimulation of the PMNs. This cellular cross-talk occurs in absence of physical binding of PMNs to EC indicating that the event of PMN adhesion and structural linkage per se does not directly transfer signals to EC whereby the permeability response is mediated. Rather, β2 integrin ligand-binding initiates through an intracellular signaling cascade PMN secretory events and release of a chemical messenger, which capacitates the PMN-dependent change in EC permeability. Further, it is demonstrated that the alteration in vascular permeability induced via this pathway is a consequence of active, tyrosine kinase-dependent responses of EC involving cytoskeletal reorganization and reversible intercellular gap formation indicating a non-lytic function in this respect of the PMN-derived factor(s).

In order to explore in more detail mechanisms by which activated PMNs may transfer signals to ECs in this process, PMN/EC interactions and adhesion-dependent engagement of β₂ integrins are simulated through antibody cross-linking of CD11/CD18. Through antibody cross-linking of β2 integrins, bypassing chemoattractant-induced cellular activation, it is demonstrated that engagement and clustering of β2 integrin receptors per se via intracellular signaling events trigger an increase in EC permeability. Since this response occurred irrespective of PMN adhesion to EC, transfer of signals between PMNs and EC cannot be ascribed to an interaction of CD11/CD18 with counter-receptors on the EC surface, but rather, must be accomplished via release of a PMN-derived messenger molecule. Such mechanism is further supported by the capacity to induce EC hyperpermeability of the cell-free supernatant obtained after CD18 cross-linking in PMNs in suspension.

### Example 2: Identification of Neutrophil-Derived HBP (HBP) as Signaling Link in Neutrophil-Induced Vascular Plasma Leakage

In Example 1, PMN adhesion through the β-2 integrin is a prerequisite for the PMN induced effects on endothelial cell permeability and that a Ca²⁺ dependent transmembrane signaling through the adhesion activated β-2 integrin induced secretion of a soluble factor(s) which directly trigger the changes in the EC barrier function.

As described in Example 2 herein, the soluble factor is determined to be human HBP. Example 2 also describes the characterization of the interaction of HBP with the contact phase system.

### MATERIALS AND METHODS

### Reagents

Medium-199, RPMI-1640 (containing L-glutamine), fetal bovine serum (FBS), trypsin-EDTA, phosphate buffered saline (PBS) and Hanks balanced salt solution (HBSS) are from Life Technologies (Gaithersburg, MD, USA). Gelatin, collagenase, penicillin, streptomycin, bovine serum albumin (BSA), Evans blue dye and FITC-conjugated phalloidin are obtained from Sigma Chemical Co. (St. Louis, MO, USA). Fluo-3/AM are obtained from Molecular Probes (Eugene, OR, USA). Biomatrix I is from Biomedical Technologies Inc. (Stoughton, MA, USA). Dextran 70 (Macrodex) and Ficoll-Paque are obtained from Pharmacia Biotech AB (Uppsala, Sweden). The basal culture medium consisted of a 1:1 mixture of M-199 and RPMI-1640 supplemented with 20% heat inactivated FBS, penicillin (100 U/ml) and streptomycin (100 µg/ml). The monoclonal antibody IB4 against the common β-chain (CD18) of β₂-integrins is provided by Dr. Claes Lundberg, Pharmacia & Upjohn (Uppsala, Sweden) by courtesy of Dr. Samuel D. Wright, Rockefeller University. F(ab')₂ fragment of goat anti-mouse IgG is from Jackson ImmunoResearch Laboratories, Inc. (West Grove, PA).

Polyclonal HBP antibodies are prepared according to the following procedure. New Zealand white rabbits are injected subcutaneously with 50 µg of human heparin-binding protein (HBP) emulsified in Freund's Complete Adjuvant (FCA), followed by three booster injections with 50 µg human HBP emulsified in Freund's Incomplete Adjuvant (FIA) every two weeks. Ten days after the last injection, the animals are bled and serum is isolated.

Monoclonal HBP antibodies are prepared according to the following procedure. RBF mice are immunized by injecting subcutaneously 20 µg of purified human HBP in FCA followed by two injections with 20 µg of human HBP in FIA. High responder mice are boosted intravenously with 20 µg of HBP and the spleen is harvested after three days. Spleen cells are fused with the myeloma Fox cell line. Supernatants are screened for anti-HBP antibody production in an HBP-specific ELISA. Positive lines are screened for binding to the intracellular part of a macrophage line by standard FACS methodology. Positive lines are cloned and retested in both assays. Monoclonal antibodies are purified by Protein A affinity chromatography.

### Endothelial cells

Human umbilical vein endothelial cells (HUVEC) are isolated from fresh umbilical cords as described by Jaffe et al., 1973, J. Clin. Invest. 52:2745-2756 ), and bovine aorta endothelial cells (BAEC) are isolated according to the method of Booyse et al., 1975, Thromb. Diath. Haemmorrh. 24:825-839) with a few modifications. Briefly, the lumen of the umbilical vein or bovine aorta is incubated with a 0.25% collagenase solution for 10 min, and detached endothelial cells washed out with PBS. Harvested cells are pelleted and resuspended in culture medium, seeded in tissue culture flasks and incubated under standard conditions. Medium is changed every 48 h until cultures have reached confluence (3-5 days). HUVEC or BAEC in the first to fifth passages are used in the experiments. Identification and purity determination of ECs are based on cobblestone morphology and characterization by factor VIII/vWf antigen (Booyse et al., 1975, Thromb. Diath. Haemmorrh. 24:825-839). Staining for factor VIII/vWf factor is homogeneous in the cells.

### Seeding onto permeable membranes

Cultured EC are detached by brief (2 min) trypsin-EDTA treatment (0.25% trypsin/0.01 % EDTA) and replated onto either 0.2 µm pore size Anopore inorganic membrane or 3.0 µm pore size polycarbonate filters (Tissue Culture inserts, 10 mm; NUNC, Roskilde, Denmark). In order to promote cellular differentiation and enhance attachment of EC, the filters are treated with 50 µl Biomatrix I (167µg/ml) and left to air-dry. EC are seeded at a density of 2 x 10⁵ cells per filter and incubated in culture medium at 37°C in a humidified atmosphere of 5% CO₂ in air. The ECs are grown to confluent monolayers, as assessed by daily microscopic observation and measurement of the electrical resistance across the monolayer.

### Measurement of transendothelial electrical resistance (TEER)

For measurement of electrical resistance across the EC monolayer, the filter insert is transferred to a resistance measurement chamber (ENDOHM-12; World Precision Instruments, Sarasota, FL, USA) modified so as to assure exact positioning of the electrodes in relation to each other and to the filter insert during repeated measurements. The chamber (lower compartment) and the filter insert (upper compartment) are filled with 2 ml and 400 µl culture medium, respectively. All measurements are made at 37°C with the equipment placed in a cell culture incubator. Direct readings of the electrical resistance are given by an ohmmeter (EVOM; World Precision Instruments, Sarasota, FL, USA) connected to the electrodes of the resistance chamber. The electrical resistance of individual EC monolayers is obtained by subtracting the resistance of the corresponding naked filter coated with Biomatrix as measured prior to seeding of the endothelial cells.

### Determination of albumin clearance

Evans blue dye-conjugated albumin (EBA) is used as a marker for macromolecular permeability of the EC monolayers. The dye at a final concentration of 0.67 mg/ml is mixed with culture medium containing 4% BSA. In experiments where albumin permeability is analyzed, the culture medium in the upper compartment is exchanged for medium containing EBA. The EBA concentration in fluid samples of the upper and lower compartment is determined spectrophotometrically through measurement of absorbency at 620 nm (Titertek Multiskan MCC; Flow Laboratories, Solna, Sweden). Albumin clearance is calculated according to the relationship: V1 = A2 x V2 x 1/A1, where V1 is the clearance volume (i.e. the theoretical volume of the apical medium cleared from albumin by diffusion to the basolateral compartment), V2 is the basolateral volume, and A1 and A2 is the absorbency of the apical and basolateral medium, respectively. The baseline clearance in absence of any stimulus, averaging 0.08 ± 0.01 µl/min, is subtracted from the clearance volume obtained in response to respective specific stimulus.

### PMN isolation and activation

Leukocyte-rich plasma, obtained from human whole blood by dextran sedimentation, is carefully layered onto Ficoll-Paque and centrifuged at 400g for 30 min. The PMN-containing pellet is resuspended and washed twice (400g, 7 min) in ice cold PBS. The PMNs are resuspended in culture medium at a final concentration of 2-5 x 10⁶ cells/ml. Purity of PMNs is > 98% and viability by Trypan blue exclusion is >95%.

Antibody cross-linking of CD18 molecules on the neutrophil surface is induced as described above. In brief, purified PMNs (5 x 10⁶/ml) are incubated with anti-CD18 mAb IB4, 10 µg/ml in HBSS for 30 min. During the incubation period, the cells are subjected to temperature transition from 4° to 37° C in order to increase surface expression of CD11/CD18. The cells are washed twice and cross-linking of CD18 is accomplished through addition of F(ab')₂ fragments of goat anti-mouse IgG (diluted 1:20). The PMNs are sedimented by centrifugation at 300g for 15 min at room temp, and the cell-free supernatant is collected. In separate experiments, antibody cross-linking of CD18 is induced after PMNs preincubated with mAb IB4 are added to EC monolayers (see below).

### Experimental procedures

Before transferring the filter inserts to the resistance measurement chamber (kept at 37° C), the medium in the insert and in the chamber is replaced with fresh culture medium (37°C) supplemented with 10 mM Hepes. HBP (25-100 µg/ml) or PMN secretion product is added either to the upper or the lower compartment and change in transendothelial electrical resistance is measured every minute until a plateau phase is reached, and thereafter at 5-min intervals. In separate experiments, PMNs (2 x 10⁶) preincubated with mAb IB4 (10 µg/ml) are added to the upper compartment (PMN:EC ratio = 10:1) and allowed to sediment for 10 min. Activation of the PMNs is induced through antibody crosslinking of leukocytic CD18 by addition of goat anti-mouse IgG F(ab')₂ diluted 1:20. In experiments where albumin permeability across the EC monolayer is studied, the medium in the insert contained EBA. During the experiment, the filter insert is moved at 5-10 min intervals to new wells containing fresh medium. All incubations are made at 37°C. After the experiment, the medium in the lower wells is aspirated and analyzed for EBA content.

### Determination of EC intracellular Ca²⁺ and F-actin distribution

The Ca²⁺ sensitive fluorescent probe fluo-3/AM is used for determination of changes in EC intracellular free Ca²⁺. Confluent HUVEC or BAEC monolayers cultured on Biomatrix I coated filters are incubated with a Fluo-3/AM solution (3µM in HBSS containing 2% FCS and 10 µM HEPES), added to both the apical and basolateral surface, for 30 min at 37° C. The monolayers are washed three times and incubated with fresh HBSS in the dark for 20 min at room temperature before use to allow complete hydrolysis of the dye ester to its calcium sensitive free acid form. EC are stimulated with HBP added to the apical surface. Changes in EC [Ca²⁺]ᵢ in response to HBP stimulation are measured through continuous registration of fluorescence intensity with a laser-scanning confocal imaging system (Insight Plus; Meridian Instruments Inc., Okemon, Michigan).

For analysis of F-actin distribution in EC in response to stimulation with HBP, confluent EC monolayers grown on Biomatrix coated coverslips are incubated with HBP or vehicle alone at 37° C for 15 min. The monolayers are washed twice in culture medium and fixed in 3.7% formaldehyde in PBS for 10 min at room temperature. EC are permeabilized with 0.2% Triton X-100 in PBS (1 min at 4° C), washed twice and stained for actin filaments with FITC-conjugated phalloidin for 20 min at 37° C. The endothelial cells are again washed three times in PBS and viewed in a laser scanning confocal microscope.

### Western blot analysis

A sample of purified HBP or PMN-secretion is added to SDS sample buffer, and boiled for 5 min. Electrophoresis is performed by applying the samples to SDS polyacrylamide gradient gels (3-10 %). Proteins are electrophoretically transferred onto a nitrocellulose membrane (Bio-Rad Laboratories, Richmond, CA) for immunoblot analysis. Additional binding sites are blocked by incubating the nitrocellulose membrane for 2 hr. in milk. HBP is detected using western blot analysis.

### RESULTS

### Activated PMN release HBP

MNs in suspension are activated through antibody cross-linking of CD18. The PMNs are centrifuged and the supernatant is collected and run on SDS-PAGE. Western blot analysis using antibodies directed against HBP recognized a prominent band of 27 kD in the PMN secretion. No specific bands are found with control rabbit IgG.

### HBP increases permeability of EC monolayers

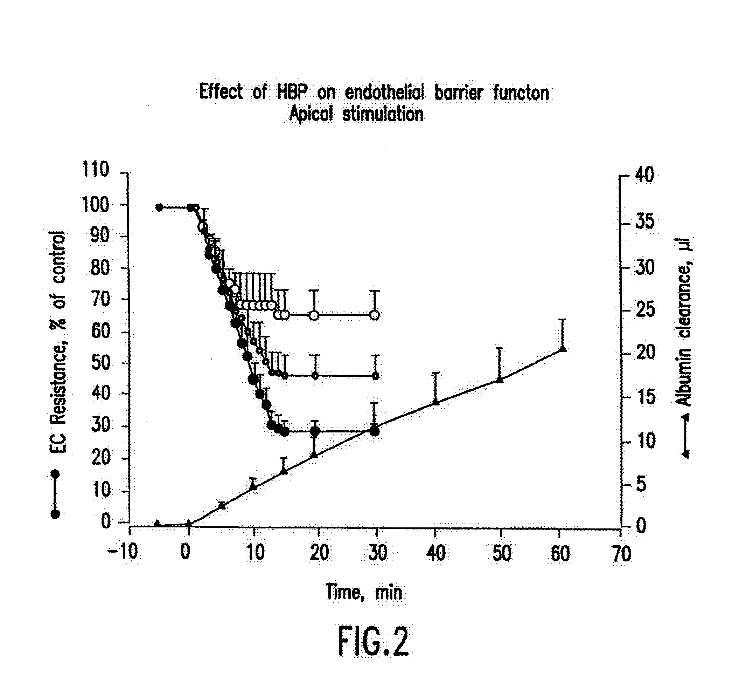

Addition of HBP (75 µg/ml) to the upper compartment elicited a marked decrease in transendothelial resistance, which is manifest within one min, reached its maximum (30% ± 3% of control) after 15 min, and so remained throughout the observation period (Figure 2). Challenge with HBP also caused an increase in EC macromolecular permeability, as reflected in a progressive rise in albumin clearance yielding a net clearance volume of 20.4 ± 3.4 µl over a 60 min period (Figure 2). The EC responses to HBP stimulation are clearly dose-dependent as illustrated by less pronounced decreases in TEER at steady state with HBP 25 and 50 µg/ml (Figure 2). However, the rate of change by which the responses occur is the same for all doses of HBP.

Stimulation of EC with HBP from the basolateral side of the monolayer (HBP added to the lower compartment) elicited similar changes in EC permeability as for apical stimulation, however at somewhat higher concentrations.

### Neutralization of HBP attenuates PMN-induced increase in EC permeability

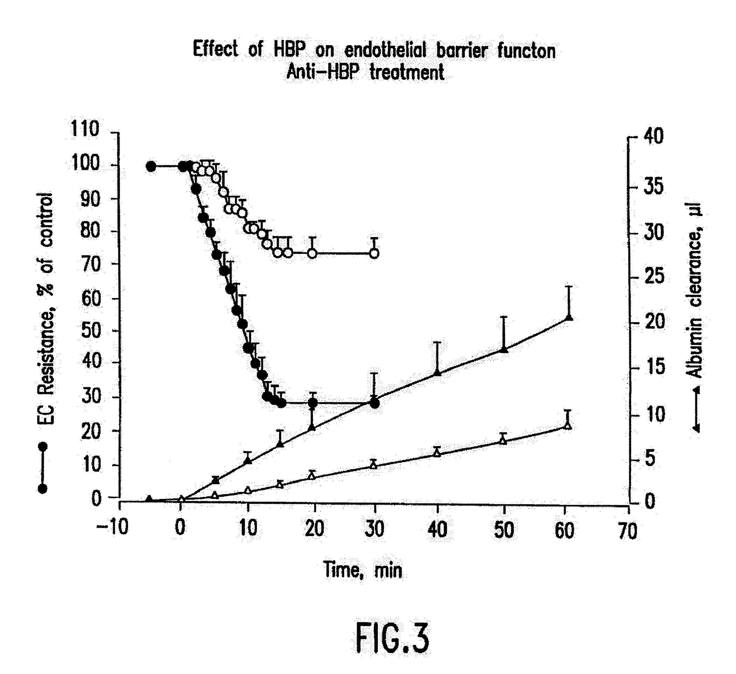

Purified IgG of a rabbit antiserum against HBP is used to neutralize the activity of the protein. Addition of HBP antibodies (50 µg/ml) to the EC monolayer prior to stimulation with HBP (75 µg/ml) markedly attenuated the evoked changes in TEER and macromolecular permeability. The change in TEER occurred with some latency and reached to 75% ± 5% of control (Figure 3), signifying an inhibition of the HBP-induced response by approximately 65 %. Similarly, the albumin efflux is inhibited by approximately 60 % to a net clearance volume of 8.64 ± 1.74 µl over 60 min (Figure 3). Treatment with non-specific rabbit IgG (50 µg/ml) is without effect on HBP-induced decrease in TEER, reaching 31% ± 5% of control in 15 min.

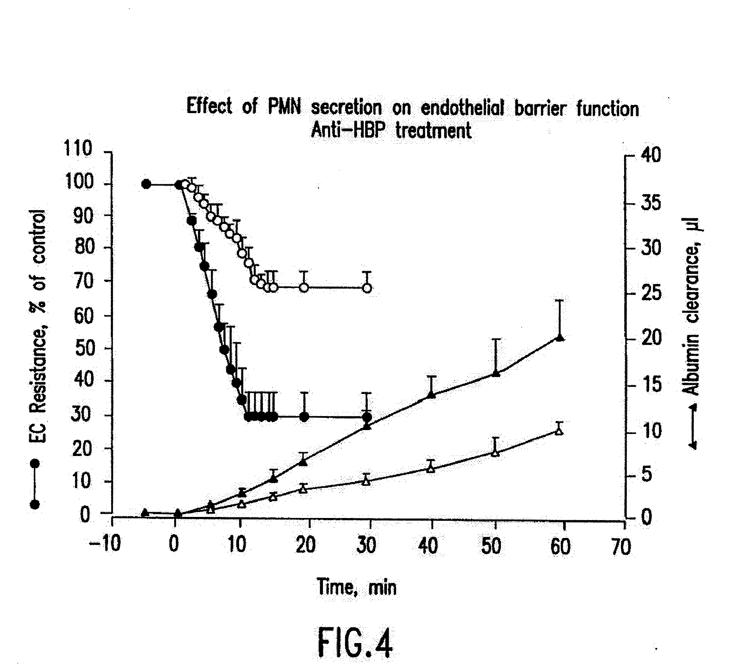

A determination is made whether antibodies against HBP could exert inhibitory actions also on changes in EC barrier function triggered by activated PMNs. PMNs in suspension are activated through antibody cross-linking of leukocytic CD18 and centrifuged. The supernatant containing PMN secretion products (see Materials and Methods) is transferred to the EC monolayer. In the presence of antibodies against HBP, the decrease in TEER and increase in macromolecular permeability are markedly suppressed as compared with the responses to stimulation with PMN secretion in absence of anti-HBP (Figure 4). Almost identical effects are obtained when antibody cross-linking of CD18 is induced after PMNs have been added to the EC monolayer and hence, the PMN secretion product directly reached the EC monolayer.

### HBP stimulation increases EC intracellular free Ca²⁺

Laser scanning confocal microscopy is employed to determine if EC responded to HBP stimulation by an increase in cytosolic Ca²⁺. Addition of HBP (75 µg/ml) to confluent HUVEC or BAEC monolayers, loaded with the Ca²⁺ sensitive fluorophore fluo-3/AM, provoked a rapid increase in fluorescence intensity, which peaked within 1 min and then gradually declined toward baseline levels. This intracellular Ca²⁺ mobilization clearly indicates an active response of EC to HBP stimulation.

### HBP stimulation induces reorganization of EC microfilaments

Confluent EC monolayers incubated with HBP (75 µg/ml) or culture medium alone for 15 min are stained for actin filaments with FITC-conjugated phalloidin and viewed in a laser scanning confocal microscope. In comparison with untreated EC, exhibiting low density of F-actin stress fibers and distinct dense peripheral bands along the cell margin, there are substantial changes in F-actin content and distribution in the HBP stimulated EC. An increased density of F-actin stress fibers spanning the cell and disruption of peripheral bands indicated reorganization of the EC cytoskeleton as a consequence of HBP stimulation.

### HBP increases vascular permeability in vivo

Topical application of HBP (75 µg/ml) to the hamster cheek pouch microvasculature results within 2 min in visible leaky spots of the fluorescent tracer of both postcapillary and larger venules. The maximum leakage response is seen at 7-10 min after challenge. The leakage then subsided slowly and disappeared completely within 30 min.

### Inhibition of Bradykinin- and HBP-Induced Increase in EC Permeability with Monoclonal Antibody MBK3 to Bradykinin

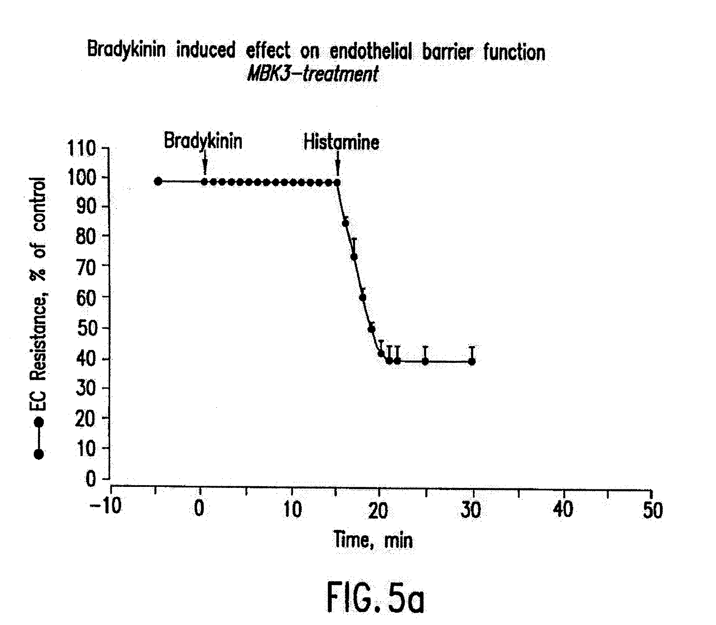

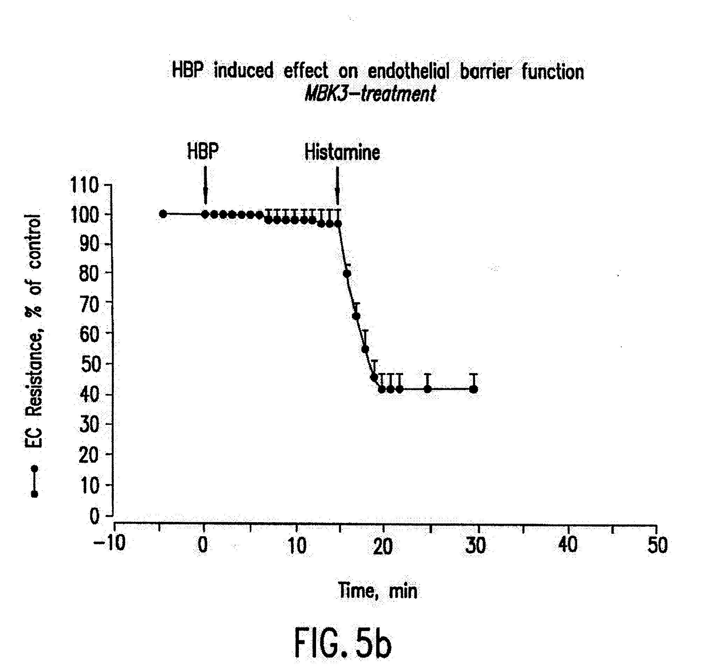

Bradykinin (100 nM; Figure 5a) or HBP (75 µg/ml; Figure 5b) is administered at time zero to the luminal side of EC monolayers pre-incubated with a monoclonal antibody to bradykinin, MBK3 (40 µg/ml) (Haasemann et al., 1991, J. Immunol. 147:3882-3892). MBK3 totally prevented the increase in EC permeability induced by either bradykinin or HBP. The specificity of the mAb effect is documented by the lack of inhibition of histamine-induced permeability change (histamine administered at 15 min after stimulation with bradykinin or HBP), N=6.

### Inhibition of HBP-induced increase in EC permeability with monoclonal antibody PKH4 to plasma kallikrein

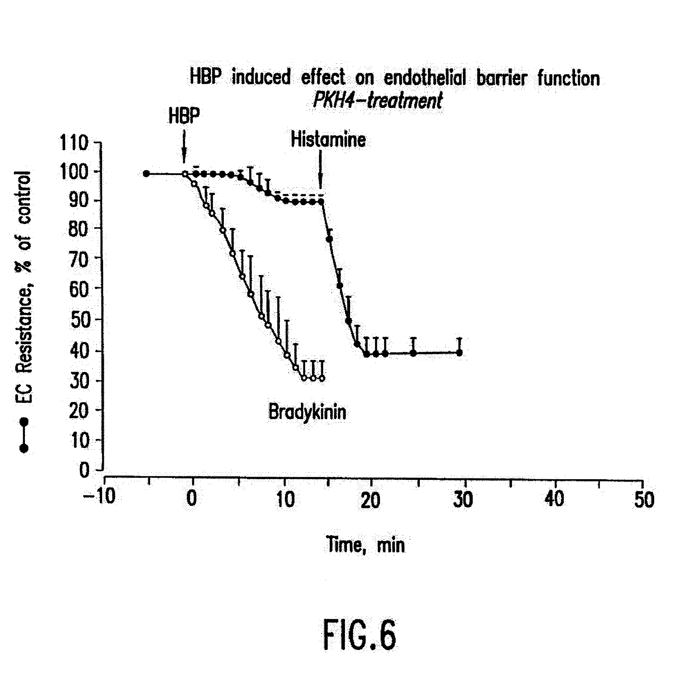

HBP (75 µg/ml; filled symbols) or bradykinin (100 nM; open symbols) is administered at time zero to the luminal side of EC monolayers pre-incubated with mAb PKH4 (40 µg/ml). The results are shown in Figure 6. PKH4 prevented the increase in EC permeability induced by HBP, but not that induced by bradykinin. PKH4-treatment did not affect the permeability change induced by histamine (histamine administered at 15 min after stimulation with HBP), N=6.

### Inhibition of HBP-induced increase in EC permeability by peptide HKH20-treatment

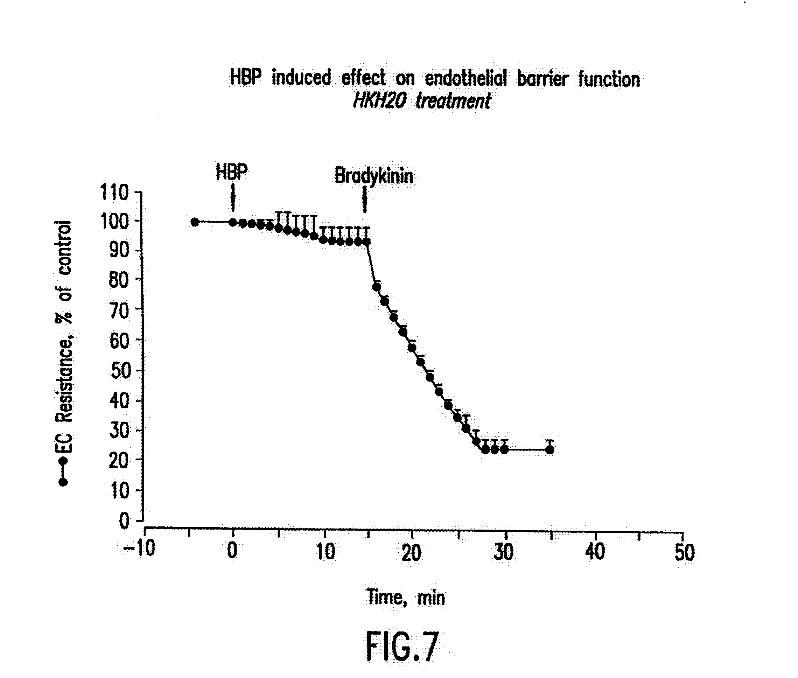

EC monolayers are incubated with HKH20 (3 µg/ml) for 30 min, which treatment displaces surface-bound H-kininogen. After wash, HBP (75 µg/ml) is administered at time zero to the luminal side of ECs followed by bradykinin (100 nM) at 15 min. HK is anchored to the EC surface by domain 5 and 6, with domain 5 being the most important. The minimum amino acid sequence required for human HK binding in domain 5 to EC is represented by a 20 amino acid peptide (HKH 20) with the following sequence: HKHGHGHHKKNKGKKNGKH (SEQ ID NO:7) (human H-kininogen 479-498). This peptide inhibits the attachment of HK to EC (Hasan et al., 1995, J. Biol. Chem. 270:19256-19261 and Herwald et al., 1996, J. Biol. Chem. 271:13040-13047). The results are shown in Figure 7. HKH20-treatment of EC prevented HBP-induced increase in EC permeability, but not the permeability change induced by subsequent administration of bradykinin, N=6.

### Inhibition of HBP-induced increase in EC permeability by peptide SDD31-treatment

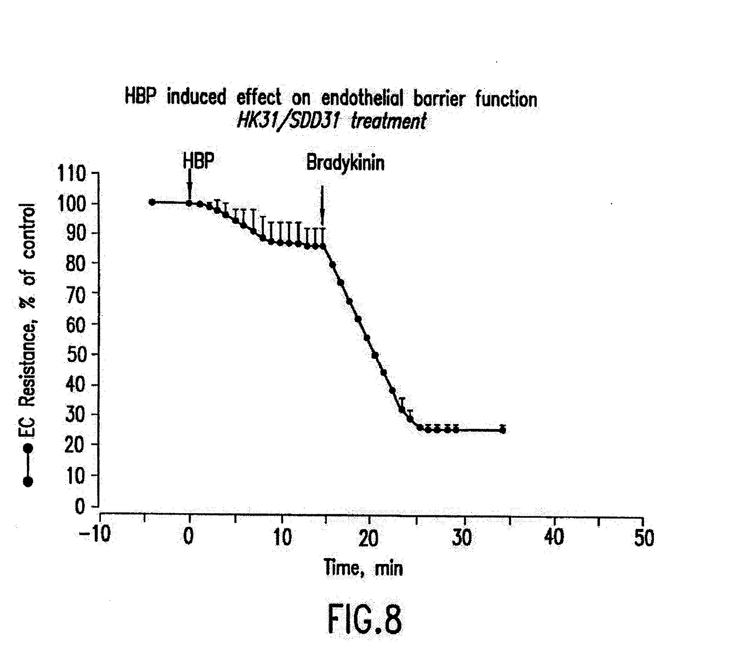

EC monolayers are incubated with SDD31 (3 µg/ml) for 30 min. SDD31, also referred to as HK31, is a peptide derived from human H-kininogen ·HK) domain 6, which blocks the binding of HK to plasma kallikrein and factor XII (Tait et al, 1986, J. Biol. Chem. 261:15396-15401 and Vogel et al., 1990, J. Biol. Chem. 265:12494-12502). It has the sequence SDDDWIP-DIQTDPNGLSFNPISDFPDTTSPK(SEQ ID NO:8) (human H-kininogen 565-595). After wash, HBP (75 µg/ml) is administered at time zero to the luminal side of EC followed by bradykinin (100 nM) at 15 min. The results are shown in Figure 8. SDD31-treatment of EC largely prevented HBP-induced increase in EC permeability. The EC response to direct stimulation with bradykinin persisted after SDD31-treatment, N=6.

### Inhibition of HBP-induced increase in EC permeability by aprotinin-treatment

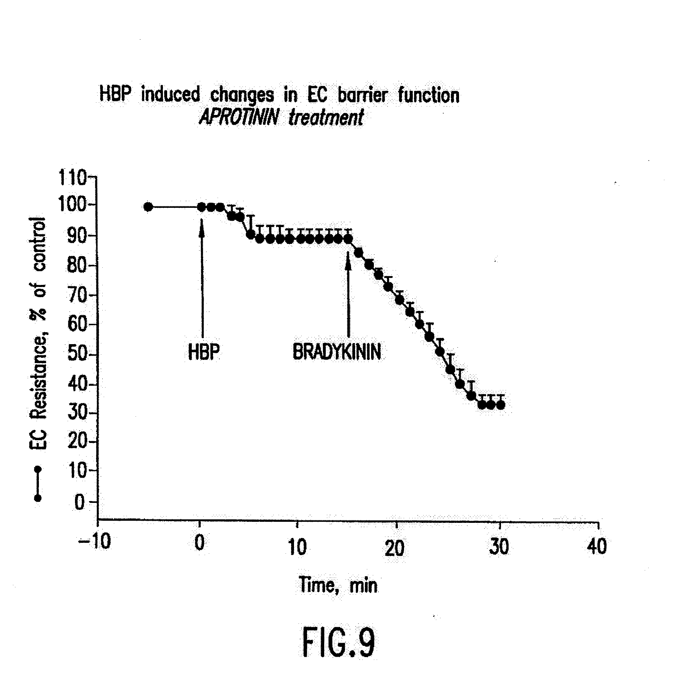

HBP (75 µg/ml) is administered at time zero to the luminal side of EC monolayers pre-incubated with aprotinin (100 µg/ml; 30 min). The results are shown in Figure 9. Aprotinin-treatment largely prevented HBP-induced increase in EC permeability, but not the permeability change induced by subsequent administration of bradykinin (100 nM), N=6.

An HBP mutant (G175N) is unable to bind aprotinin due to the mutation but the three dimensional structure and the special surface charge distribution is unaltered. When tested in the leakage model, this mutant induces bradykinin release and increase in EC permeability. However, addition of aprotinin has no inhibitory effect. A similar result is obtained with porcine HBP which is very homologous to human HBP but is unable to bind aprotinin due to the presence of an R in the bottom of the degenerated catalytic cleft.

### Example 3: Two Mutants of Heparin-Binding Protein

Two mutants of human HBP, [R23S,F25E]HBP and [G175Q]HBP, have been produced to investigate structure-function relationships of residues in the putative lipid A/LPS binding site and BPTI (bovine pancreatic trypsin inhibitor) binding site. The G175Q mutation does not alter the structure of the protein, but the ability to bind BPTI has been eliminated, and the mutant mediates only a very limited stimulation of the lipopolysaccharide (LPS) induced cytokine-release from human monocytes. The lipid A/LPS binding property is comparable to that of native HBP. The R23S,F25E mutations do not affect the binding of lipid A/LPS and BPTI or the lipopolysaccharide (LPS) induced cytokine-release from human monocytes.

### MATERIALS AND METHODS

### Construction of mutant proteins

Pwo DNA polymerase is from Boehringer Mannheim, *Bam*H1 and T4 DNA ligase are from New England Biolabs, *spodoptera frugiperda* (*sf*9) cells and pVL1393 transferplasmid are from Invitrogen, TC100 and SF900-II are produced at Novo Nordisk A/S, Foetal calf serum (FCS) is from GibcoBRL. All other chemicals are of the finest grade available.

### [R23S,F25E]HBP mutant

The [R23S,F25E]HBP mutant is constructed using the PCR overlap-extension technique (Ho, S. N., Hunt, H. D., Horton, R. M., Pullen, J. K., and Pease, L. R. (1989) *Gene (Amst.)77*: 51-59). Two partially overlapping mutated fragments are prepared by PCR using oligonucleotides 1+2 and 3+6 and the resulting fragments are joined using oligonucleotide 1+6 (Table 1). Native human HBP cDNA is used as template for the PCR reaction. The resulting cDNA encoded the 19 amino acid pro-peptide, the 222 amino acid mature part and a three amino acids C-terminal extension. *Bam*H1 sites are incorporated on each side of the mutated cDNA for cloning purposes. The mutated fragment is ligated into the pVL1393 transfer plasmid (Invitrogen). DNA for transfection is prepared using the Qiagen midiprep kits. Following purification, the insert is sequenced.

**Table 1: Oligonucleotides used for construction of the [R23S,F25E]HBP and [G175Q]HBP mutants**

| No. | Sequence |
|---|---|
| 1. | 5'-CCGGGGATCCGATGACCCGGCTGACAGTCCTGG-3' (SEQ ID NO:9) |
| 2. | 5'-GGCACCCCCGCACTCGTGGCTGCCTTGATTCTG-3' (SEQ ID NO:10) |
| 3. | 5'-CAGAATCAAGGCAGCCACGAGTGCGGGGGTGCC-3' (SEQ ID NO:11) |
| 4. | 5'-GAGGGGGGTGCCCTGGTCCCCATTGCA-3' (SEQ ID NO:12) |
| 5. | 5'-TGCAATGGGGACCAGGGCACCCCCCTC-3' (SEQ ID NO:13) |
| 6. | 5'-CCGGGGATCCAACTAGGCTGGCCCCGGTCCCGG-3' (SEQ ID NO:14) |

### [G175Q]HBP mutant.

This mutant is in general constructed as described above. Two partially overlapping mutated fragments are prepared by PCR using oligonucleotides 1+4 and 5+6 and the resulting fragments are joined using oligonucleotides 1+6 (Table 1).

### Expression and purification

sf9 insect cells are maintained in TC100 medium supplied with 10% FCS. Prior to infection, the medium is changed to serum-free SF900-II. Expression of both mutants in serum-free medium and purification of the mutants from the culture supernatant is performed as previously described for native human HBP (Rasmussen, P. B., Bjørn, S., Hastrup, S., Nielsen, P. F., Norris, K., Thim, L., Wiberg, F. C., and Flodgaard, H. (1996) *FEBS Lett. 390*: 109-112).

### Assay of LPS induced IL-6 release from monocytes

Human monocytes are isolated and treated as described (Rasmussen, P. B., Bjørn, S., Hastrup, S., Nielsen, P. F., Norris, K., Thim, L., Wiberg, F. C., and Flodgaard, H. (1996) *FEBS Lett. 390:* 109-112), with the exception that 1 % non-essential amino acids is excluded from the medium.

### Assay of ³H-LPS binding to HBP

The affinity for lipopolysaccharide (³H-LPS) is measured by scintillation proximity assay technology (SPA) as described (Linde, V., Bjørn, S., Kastrup, J. S., and Flodgaard, H., 2000, Biotechniques 28:218-222). All samples are repeated in triplicates.

### Assay of ¹²⁵I-BPTI binding to HBP

The affinity for BPTI is assessed using a SPA assay essentially as described for the LPS assay (Linde, V., Bjørn, S., Kastrup, J. S., and Flodgaard, H. , 2000, Biotechniques 28:218-222) with a few modifications. ³H-LPS is exchanged for ¹²⁵I-BPTI (prepared by Novo Nordisk A/S) and the buffer is changed to PBS (20 mM phosphate pH 7.4 and 150 mM NaCl) including 0.05 % BSA and 0.05 % Triton X-1 00. The relative affinities of HBP and the mutants are determined by performing competition studies with fixed, increasing concentrations of unlabeled BPTI.

### RESULTS

### Biological characterisation

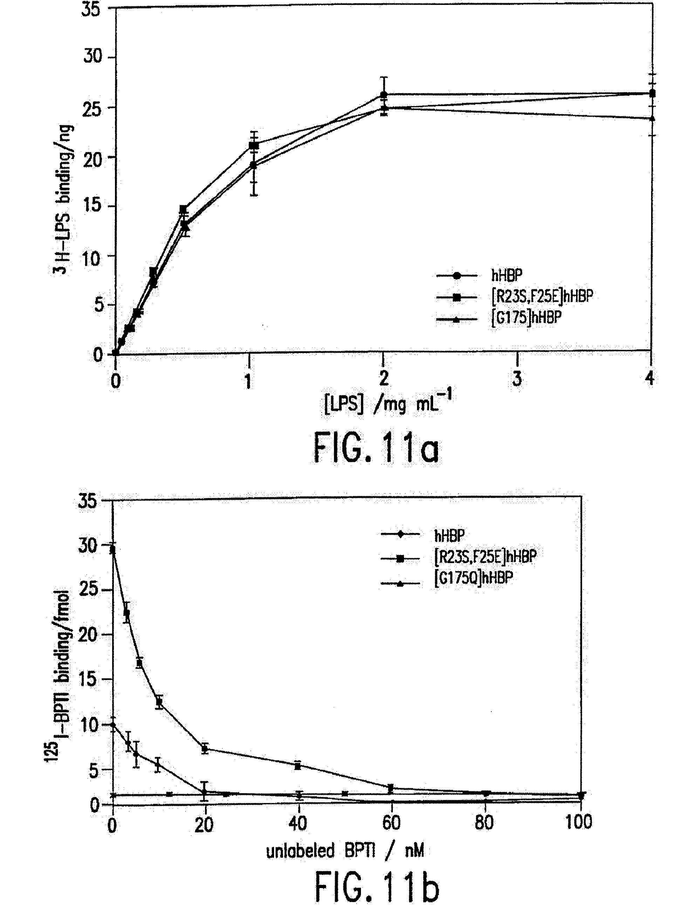

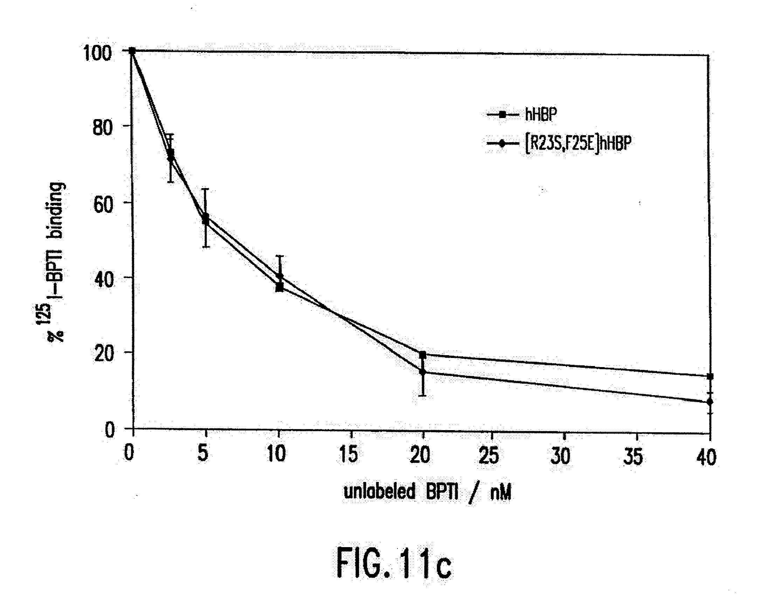

The biological activity of the two mutants are compared to native HBP with respect to their ability to enhance, in a dose-dependent manner, the LPS-induced II-6 release from human monocytes *in vitro*. The LPS affinity of the two mutants and of HBP are measured in a scintillation proximity assay (SPA) (Linde, V., Bjørn, S., Kastrup, J. S., and Flodgaard, H., 2000, Biotechniques 28:218-222). Both mutants bind ³H-LPS in a saturable manner with similar apparent *K*_{d} (approximately 0.7 g/mL) as observed for native HBP (Figure 11 a). Furthermore, the affinity for BPTI is measured for both mutants and for native HBP in a similar scintillation proximity assay, where ¹²⁵I-BPTI is used as ligand for HBP. The [R23S,F25E]HBP mutant as well as native HBP binds BPTI whereas no binding is observed for the [G175Q]HBP mutant (Figure 11 b). Competition studies performed by addition of fixed, increasing concentrations of unlabeled BPTI showed that ¹²⁵I-BPTI binds to HBP and [R23S,F25E]HBP with similar apparent IC₅₀values of approximately 7 nM. As observed in Figure 11c, [R23S,F25E]HBP apparently has a larger binding capacity as compared to HBP. This difference is probably due to an ability of [R23S,F25E]HBP to bind different forms of the iodinated BPTI used. However, the larger binding capacity does not influence the affinity of ¹²⁵I-BPTI. In addition, the ability of ³H-LPS to displace ¹²⁵I-BPTI bound to native HBP, and *vice versa*, has been investigated by competition experiments: competition for ¹²⁵I-BPTI binding by adding up to 5 µg/mL unlabeled LPS and competition for ³H-LPS binding by adding up to 50 mM unlabeled BPTI. In both situations no inhibitory effects are observed, indicating that the LPS and BPTI binding sites are located at different parts of the HBP molecule.

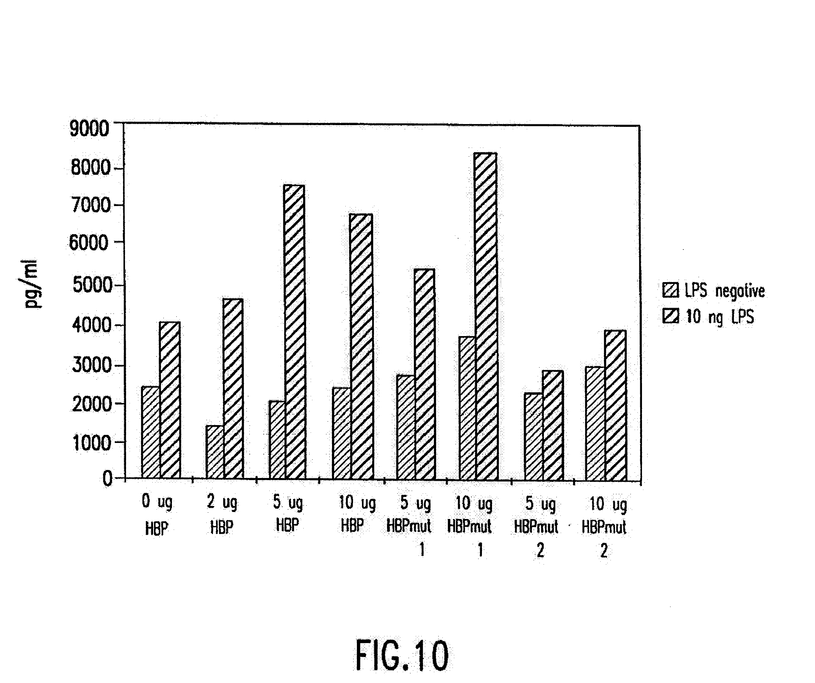

The biological *in vitro* activity assay data show that [G175Q]HBP, contrary to [R23S,F25E]HBP and native HBP, mediates only a very limited stimulation of the lipopolysaccharide (LPS) induced cytokine-release from human monocytes (Figure 10).

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims.

Various references are cited herein, the disclosures of which are incorporated by reference in their entireties.

## Claims

**1.** Use of a mammalian HBP antagonist for the preparation of a medicament for preventing or treating a disorder resulting from release of bradykinin in a mammal wherein said mammal produces HBP that binds to an HBP antagonist.

**2.** The use according to claim 1, in which the HBP antagonist is present in an amount of from about 10 mg to about 1 g per unit dosage form.

**3.** The use according to claim 1, in which the HBP antagonist is present in an amount of about 0.1-100 mg/kg body weight.

**4.** The use according to claim 1, in which the HBP antagonist is present in an amount of about 0.5-50 mg/kg body weight.

**5.** The use according to claim 1, in which the HBP antagonist is present in an amount of about 1-25 mg/kg body weight.

**6.** The use according to claim 1, in which the disorder is selected from the group consisting of systemic inflammatory response syndrome, ischemia reperfusion, anaphylaxis and allograft rejection.

**7.** The use according to claim 1, in which the disorder is adult respiratory distress syndrome.

**8.** Use of aprotinin or an analog thereof for the preparation of a medicament for decreasing release of bradykinin in a mammal wherein said mammal produces HBP that binds to aprotinin or an analog thereof.

**9.** The use according to claim 8 wherein said aprotinin analog is an aprotinin variant containing at least one mutation in the 15-19 position.

**10.** Use of aprotinin or an analog thereof, for the preparation of a medicament useful for the treatment of systemic inflammatory response syndrome, ischemia reperfusion, anaphylaxis and allograft rejection in a patient having a HBP that binds to aprotinin or an analog thereof.

**11.** Use of aprotinin or analog thereof, for the preparation of a medicament useful for the treatment of adult respiratory distress syndrome in a patient having a HBP that binds to aprotinin or an analog thereof.

**12.** Use of a monoclonal antibody that binds at least one epitope of HBP wherein said epitope binds to prekallikrein-H-kininogen complex and activates bradykinin for the preparation of a medicament for preventing or treating a disorder resulting from release of bradykinin in a mammal wherein said mammal produces HBP that binds to said monoclonal antibody that binds at least one epitope of HBP wherein said epitope binds to prekallikrein-H-kininogen complex and activates bradykinin.

**13.** The use according to claim 12, wherein said mammal is a human patient and said HBP is human HBP.

**14.** The use according to claim 12, wherein said monoclonal antibody is a human monoclonal antibody.

**15.** The use according to claim 12 for the preparation of a medicament useful for the treatment of systemic inflammatory response syndrome, ischemia reperfusion, anaphylaxis and allograft rejection.

**16.** The use according to claim 12 for the preparation of a medicament useful for the treatment of adult respiratory distress syndrome.

**17.** A method of identifying an antagonist of HBP, said method comprising (a) culturing endothelial cells in the presence of HBP and in the presence and absence of a substance suspected of being said antagonist and (b) detecting any effect of said substance on permeability of endothelial cells, wherein decreased permeability of said endothelial cells as compared to permeability of said cells when incubated in the presence of HBP without said substance indicates that said substance is an antagonist.

**18.** The method according to claim 17, wherein in step (a) endothelial cells are first cultured in the presence of HBP and permeability of said cells are measured and then said cells are subsequently cultured in the presence of said substance.

**19.** The method according to claim 17, wherein in step (a), endothelial cells are first cultured in the presence of said substance and then said cells are subsequently cultured in the presence of said HBP.

**20.** The method according to claim 17, wherein one sample of endothelial cells are cultured in the presence of HBP and a second sample is cultured in the presence of HBP and said substance.

**21.** The method according to claim 17, in which the mammalian HBP is a human HBP.

**22.** A method for identifying a HBP antagonist comprising (a) incubating HBP with a first substance that interacts with HBP and a second substance suspected of being a HBP antagonist and (b) detecting any effect of said second substance suspected of being an antagonist on interaction of HBP with said first substance, wherein decreased interaction between said HBP with said first substance is an indication that said second substance is an HBP antagonist.

**23.** A method of identifying a monoclonal antibody that binds to at least one epitope on native HBP, wherein said epitope binds to prekallikrein-H-kininogen complex and activates release of bradykinin, said method comprising (a) culturing endothelial cells in the presence of HBP and in the presence and absence of a substance suspected of being said antagonist and (b) detecting any effect of said substance on permeability of endothelial cells, wherein decreased permeability of said endothelial cells as compared to permeability of said cells when incubated in the presence of HBP without said substance indicates that said monoclonal antibody binds to at least one epitope on said HBP.

**24.** The method according to claim 23, wherein said native HBP is a human HBP.

**25.** A method of identifying a monoclonal antibody that binds to at least one epitope on native HBP, wherein said epitope binds to prekallikrein-H-kininogen complex and activates release of bradykinin, said method comprising (a) incubating a prekallikrein-H-kininogen complex in the presence of HBP and in the presence and absence of a monoclonal antibody suspected of binding to at least one epitope on said HBP and (b) detecting any effect of said antibody on release of bradykinin, decreased bradykinin release indicating that said antibody binds to at least one epitope on said HBP.

**26.** The method according to claim 25, wherein the prekallikrein-H-kininogen complex is attached to a solid support.

**27.** The method according to claim 25, which further comprises incubating the mixture of step (a) in the presence of endothelial cells.

**28.** The method according to claim 25, which further comprises incubating the mixture of step (a) in the presence of platelets.

**29.** The method of claim 25, wherein the native HBP is native human HBP.

**30.** The method according to claim 25, wherein the release of bradykinin is detected by an immunoassay.

**31.** A method for determining if a mammal produces HBP that binds to an HBP antagonist comprising (a) isolating HBP or cells or tissue producing HBP from a patient; (b) incubating said HBP or cells or tissue producing HBP with a substance, tissue, cells or component thereof which interacts with HBP and said HBP antagonist and (c) detecting an effect of said HBP antagonist on the interaction of HBP with said substance, tissue, cells or components thereof, decreased interaction indicating that said HBP binds to said HBP antagonist.

**32.** The method according to claim 31, wherein said HBP is first incubated with said antagonist, then subsequently incubated with a substance, tissue, cells or component thereof which interacts with HBP.

**33.** The method according to claim 31, wherein said HBP is first incubated with a substance, tissue, cells or component thereof which interacts with HBP and then with said antagonist.

**34.** A test kit comprising (a) HBP antagonist; (b) HBP or cell producing HBP and (c) a substance, tissue, cells or component thereof which interact with HBP.

**35.** The test kit according to claim 34, wherein the HBP is labeled.

**36.** The test kit according to claim 34, wherein the test kit comprises a substance which interacts with HBP.

**37.** The test kit according to claim 36, wherein the substance is H-kininogen.

**38.** The test kit according to claim 36, wherein the substance is labeled.

**39.** The test kit according to claim 36, which further comprises beads.

**40.** A method for determining if a mammal produces HBP that binds to a monoclonal antibody that binds to at least one epitope on native HBP, wherein said epitope binds to prekallikrein-H-kininogen complex and activates release of bradykinin, comprising (a) isolating HBP or cells or tissue producing HBP from said mammal; (b) culturing said HBP or cells or tissue producing HBP with endothelial cells in the presence or absence of said antibody and (c) detecting any effect of said antibody on permeability of endothelial cells, decreased permeability indicating that said HBP binds to said antibody.

**41.** The method according to claim 40, in which the mammal is a human patient.

**41.** The method according to claim 40, wherein the native HBP is native human HBP.

**42.** A method for determining if a mammal produces HBP that binds to a monoclonal antibody that binds to at least one epitope on native HBP, wherein said epitope binds to prekallikrein-H-kininogen complex and activates release of bradykinin, comprising (a) isolating HBP or cells or tissue producing HBP from said mammal; (b) incubating said HBP or cells or tissue producing HBP with prekallikrein-kininogen complex and (c) detecting any effect of said HBP on the release of bradykinin from the prekallikrein-H-kininogen complex, decreased release of bradykinin indicating that said HBP binds to said antibody.

**43.** A test kit comprising (a) a monoclonal antibody that binds to an epitope of HBP (b) native human HBP and (c) a prekallikrein-H-kininogen complex attached to a solid support.


SEQUENCE LISTING

<110> NOVO NORDISK A/S

<120> Inhibition Of Bradykinin Release

<130> 5694-WO,JWKi

<150> 60/132,748
<151> 1999-04-29

<150> PA 1999 00613
<151> 1999-05-06

<150> 60/157,384
<151> 1999-10-01

<150> PA 1999 01402
<151> 1999-10-01

<160> 14

<170> FastSEQ for Windows Version 3 0

<210> 1
<211> 225
<212> PRT
<213> Homo sapiens

<400> 1
Ile Val Gly Gly Arg Lys Ala Arg Pro Arg Gln Phe Pro Phe Leu Ala
 1               5                  10                  15
Ser Ile Gln Asn Gln Gly Arg His Phe Cys Gly Gly Ala Leu Ile His
            20                  25                  30
Ala Arg Phe Val Met Thr Ala Ala Ser Cys Phe Gln Ser Gln Asn Pro
        35                  40                  45
Gly Val Ser Thr Val Val Leu Gly Ala Tyr Asp Leu Arg Arg Arg Glu
    50                  55                  60
Arg Gln Ser Arg Gln Thr Phe Ser Ile Ser Ser Met Ser Glu Asn Gly
65                  70                  75                  80
Tyr Asp Pro Gln Gln Asn Leu Asn Asp Leu Met Leu Leu Gln Leu Asp
                85                  90                  95
Arg Glu Ala Asn Leu Thr Ser Ser Val Thr Ile Leu Pro Leu Pro Leu
            100                 105                 110
Gln Asn Ala Thr Val Glu Ala Gly Thr Arg Cys Gln Val Ala Gly Trp
        115                 120                 125
Gly Ser Gln Arg Ser Gly Gly Arg Leu Ser Arg Phe Pro Arg Phe Val
    130                 135                 140
Asn Val Thr Val Thr Pro Glu Asp Gln Cys Arg Pro Asn Asn Val Cys
145                 150                 155                 160
Thr Gly Val Leu Thr Arg Arg Gly Gly Ile Cys Asn Gly Asp Gly Gly
                165                 170                 175
Thr Pro Leu Val Cys Glu Gly Leu Ala His Gly Val Ala Ser Phe Ser
            180                 185                 190
Leu Gly Pro Cys Gly Arg Gly Pro Asp Phe Phe Thr Arg Val Ala Leu
        195                 200                 205

Phe Arg Asp Trp Ile Asp Gly Val Leu Asn Asn Pro Gly Pro Gly Pro
    210                 215                 220
Ala
225

<210> 2
<211> 678
<212> DNA
<213> Homo sapıens

<400> 2
atcgttggcg gccggaaggc gaggccccgc cagttcccgt tcctggcctc cattcagaat      60
caaggcaggc acttctgcgg gggtgccctg atccatgccc gcttcgtgat gaccgcggcc     120
agctgcttcc aaagccagaa ccccggggtt agcaccgtgg tgctgggtgc ctatgacctg     180
aggcggcggg agaggcagtc ccgccagacg ttttccatca gcagcatgag cgagaatggc     240
tacgaccccc agcagaacct gaacgacctg atgctgcttc agctggaccg tgaggccaac     300
ctcaccagca gcgtgacgat actgccactg cctctgcaga acgccacggt ggaagccggc     360
accagatgcc aggtggccgg ctgggggagc cagcgcagtg gggggcgtct ctcccgtttt     420
cccaggttcg tcaacgtgac tgtgaccccc gaggaccagt gtcgccccaa caacgtgtgc     480
accggtgtgc tcacccgccg cggtggcatc tgcaatgggg acgggggcac ccccctcgtc     540
tgcgagggcc tggcccacgg cgtggcctcc ttttccctgg ggccctgtgg ccgaggccct     600
gacttcttca cccgagtggc gctcttccga gactggatcg atggcgtttt aaacaatccg     660
ggaccggggc cagcctag                                                   678

<210> 3
<211> 698
<212> DNA
<213> Homo sapıens

<400> 3
ggctccagcc cccttttgga catcgttggc ggccggaagg cgaggccccg ccagttcccg      60
tcctggcctc cattcagaat caaggcaggc acttctgcgg gggtgccctg atccatgccc     120
gcttcgtgat gaccgcggcc agctgcttcc aaagccagaa ccccggggtt agcaccgtgg     180
tgctgggtgc ctatgacctg aggcggcggg agaggcagtc ccgccagacg ttttccatca     240
gcagcatgag cgagaatggc tacgaccccc agcagaacct gaacgacctg atgctgcttc     300
agctggaccg tgaggccaac ctcaccagca gcgtgacgat actgccactg cctctgcaga     360
acgccacggt ggaagccggc accagatgcc aggtggccgg ctgggggagc cagcgcagtg     420
gggggcgtct ctcccgtttt cccaggttcg tcaacgtgac tgtgaccccc gaggaccagt     480
gtcgccccaa caacgtgtgc accggtgtgc tcacccgccg cggtggcatc tgcaatgggg     540
acgggggcac ccccctcgtc tgcgagggcc tggcccacgg cgtggcctcc ttttccctgg     600
ggccctgtgg ccgaggccct gacttcttca cccgagtggc gctcttccga gactggatcg     660
atggcgtttt aaacaatccg ggaccggggc cagcctag                             698

<210> 4
<211> 232
<212> PRT
<213> Homo sapıens

<400> 4
   Gly Ser Ser Pro Leu Leu Asp Ile Val Gly Gly Arg Lys Ala Arg Pro
   1               5                   10                  15
   Arg Gln Phe Pro Phe Leu Ala Ser Ile Gln Asn Gln Gly Arg His Phe
               20                  25                  30
   Cys Gly Gly Ala Leu Ile His Ala Arg Phe Val Met Thr Ala Ala Ser
           35                  40                  45
   Cys Phe Gln Ser Gln Asn Pro Gly Val Ser Thr Val Val Leu Gly Ala
       50                  55                  60
   Tyr Asp Leu Arg Arg Arg Glu Arg Gln Ser Arg Gln Thr Phe Ser Ile
   65                  70                  75                  80
   Ser Ser Met Ser Glu Asn Gly Tyr Asp Pro Gln Gln Asn Leu Asn Asp
                   85                  90                  95
   Leu Met Leu Leu Gln Leu Asp Arg Glu Ala Asn Leu Thr Ser Ser Val
               100                 105                 110
   Thr Ile Leu Pro Leu Pro Leu Gln Asn Ala Thr Val Glu Ala Gly Thr
           115                 120                 125
   Arg Cys Gln Val Ala Gly Trp Gly Ser Gln Arg Ser Gly Gly Arg Leu
       130                 135                 140
   Ser Arg Phe Pro Arg Phe Val Asn Val Thr Val Thr Pro Glu Asp Gln
   145                 150                 155                 160
   Cys Arg Pro Asn Asn Val Cys Thr Gly Val Leu Thr Arg Arg Gly Gly
                   165                 170                 175
   Ile Cys Asn Gly Asp Gly Gly Thr Pro Leu Val Cys Glu Gly Leu Ala
               180                 185                 190
   His Gly Val Ala Ser Phe Ser Leu Gly Pro Cys Gly Arg Gly Pro Asp
           195                 200                 205
   Phe Phe Thr Arg Val Ala Leu Phe Arg Asp Trp Ile Asp Gly Val Leu
       210                 215                 220
   Asn Asn Pro Gly Pro Gly Pro Ala
   225                 230

<210> 5
<211> 756
<212> DNA
<213> Homo sapiens

<400> 5
atgacccggc tgacagtcct ggccctgctg gctggtctgc tggcgtcctc gagggccggc      60
tccagccccc ttttggacat cgttggcggc cggaaggcga ggccccgcca gttcccgttc     120
ctggcctcca ttcagaatca aggcaggcac ttctgcgggg gtgccctgat ccatgcccgc     180
ttcgtgatga ccgcggccag ctgcttccaa agccagaacc ccggggttag caccgtggtg     240
ctgggtgcct atgacctgag gcggcgggag aggcagtccc gccagacgtt ttccatcagc     300
agcatgagcg agaatggcta cgacccccag cagaacctga acgacctgat gctgcttcag     360
ctggaccgtg aggccaacct caccagcagc gtgacgatac tgccactgcc tctgcagaac     420
gccacggtgg aagccggcac cagatgccag gtggccggct gggggagcca gcgcagtggg     480
gggcgtctct cccgttttcc caggttcgtc aacgtgactg tgacccccga ggaccagtgt     540
cgccccaaca acgtgtgcac cggtgtgctc acccgccgcg gtggcatctg caatggggac     600
gggggcaccc ccctcgtctg cgagggcctg gcccacggcg tggcctcctt ttccctgggg     660
ccctgtggcc gaggccctga cttcttcacc cgagtggcgc tcttccgaga ctggatcgat     720
ggcgttttaa acaatccggg accggggcca gcctag                               756

<210> 6
<211> 251
<212> PRT
<213> Homo sapiens

<400> 6
Met Thr Arg Leu Thr Val Leu Ala Leu Leu Ala Gly Leu Leu Ala Ser
1               5                   10                  15
Ser Arg Ala Gly Ser Ser Pro Leu Leu Asp Ile Val Gly Gly Arg Lys
            20                  25                  30
Ala Arg Pro Arg Gln Phe Pro Phe Leu Ala Ser Ile Gln Asn Gln Gly
        35                  40                  45
Arg His Phe Cys Gly Gly Ala Leu Ile His Ala Arg Phe Val Met Thr
    50                  55                  60
Ala Ala Ser Cys Phe Gln Ser Gln Asn Pro Gly Val Ser Thr Val Val
65                  70                  75                  80
Leu Gly Ala Tyr Asp Leu Arg Arg Arg Glu Arg Gln Ser Arg Gln Thr
                85                  90                  95

Phe Ser Ile Ser Ser Met Ser Glu Asn Gly Tyr Asp Pro Gln Gln Asn
            100                 105                 110
Leu Asn Asp Leu Met Leu Leu Gln Leu Asp Arg Glu Ala Asn Leu Thr
        115                 120                 125
Ser Ser Val Thr Ile Leu Pro Leu Pro Leu Gln Asn Ala Thr Val Glu
    130                 135                 140
Ala Gly Thr Arg Cys Gln Val Ala Gly Trp Gly Ser Gln Arg Ser Gly
145                 150                 155                 160
Gly Arg Leu Ser Arg Phe Pro Arg Phe Val Asn Val Thr Val Thr Pro
                165                 170                 175
Glu Asp Gln Cys Arg Pro Asn Asn Val Cys Thr Gly Val Leu Thr Arg
            180                 185                 190
Arg Gly Gly Ile Cys Asn Gly Asp Gly Gly Thr Pro Leu Val Cys Glu
        195                 200                 205
Gly Leu Ala His Gly Val Ala Ser Phe Ser Leu Gly Pro Cys Gly Arg
    210                 215                 220
Gly Pro Asp Phe Phe Thr Arg Val Ala Leu Phe Arg Asp Trp Ile Asp
225                 230                 235                 240
Gly Val Leu Asn Asn Pro Gly Pro Gly Pro Ala
                245                 250

<210> 7
<211> 19
<212> PRT
<213> Homo sapiens

<400> 7
His Lys His Gly His Gly His His Lys Lys Asn Lys Gly Lys Lys Asn
1               5                   10                  15
Gly Lys His

<210> 8
<211> 31
<212> PRT
<213> Homo sapiens

<400> 8
Ser Asp Asp Asp Trp Ile Pro Asp Ile Gln Thr Asp Pro Asn Gly Leu
1               5                   10                  15
Ser Phe Asn Pro Ile Ser Asp Phe Pro Asp Thr Thr Ser Pro Lys
            20                  25                  30

<210> 9
<211> 33
<212> DNA
<213> Artificial Sequence

<220>
<223> pcr primers

<400> 9
ccggggatcc gatgacccgg ctgacagtcc tgg 33

<210> 10
<211> 33
<212> DNA
<213> Artificial Sequence

<220>
<223> pcr primers

<400> 10
ggcacccccg cactcgtggc tgccttgatt ctg 33

<210> 11
<211> 33
<212> DNA
<213> Artificial Sequence

<220>
<223> pcr primers

<400> 11
cagaatcaag gcagccacga gtgcgggggt gcc 33
<210> 12
<211> 27
<212> DNA
<213> Artificial Sequence

<220>
<223> pcr primers

<400> 12
gagggggtg ccctggtccc cattgca 27

<210> 13
<211> 27
<212> DNA
<213> Artificial Sequence

<220>
<223> pcr primers

<400> 13
tgcaatgggg accagggcac ccccctc 27

<210> 14
<211> 33
<212> DNA
<213> Artificial Sequence

<220>
<223> pcr primers

<400> 14
ccggggatcc aactaggctg gccccggtcc cgg 33